# EUROPÄISCHE PATENTSCHRIFT

(11) **EP 2 120 890 B1**
(45) Veröffentlichungstag und Bekanntmachung des Hinweises auf die Patenterteilung: **31.08.2011**
(21) Anmeldenummer: 07856610.6
(22) Anmeldetag: 12.12.2007
(51) Int. Cl.: A61K 9/50

(54) **BESCHICHTETE PELLETS**
COATED PELLETS
PILULES ENROBÉES

(30) Priorität: 14.12.2006 DE 102006059510
(43) Veröffentlichungstag der Anmeldung: 25.11.2009
(73) Patentinhaber: Grünenthal GmbH, 52078 Aachen (DE)
(72) Erfinder: ZIEGLER, Iris, 86356 Neusäss (DE); JACOBS, Irwin Dr., Defiance, MO 63341 (US)
(74) Vertreter: Bülle, Jan
(86) Internationale Anmeldenummer: PCT/EP2007/010862
(87) Internationale Veröffentlichungsnummer: WO 2008/071407

(56) Entgegenhaltungen:
- WO-A-01/80831
- WO-A-2005/023216
- WO-A-2006/069920
- DE-A1-102005 019 458
- GB-A- 1 594 102
- US-A- 4 675 236

## Beschreibung

Die Erfindung betrifft beschichtete Pellets, Verfahren zu deren Herstellung und deren Verwendung.

Zahlreiche pharmazeutische Wirkstoffe haben einen bitteren Geschmack, z.B. zahlreiche Antibiotika, insbesondere Azithromycin, Cefixim, Cefdinir, Cefpodoxim, Cefuroxim und Clarithromycin.

Üblicherweise werden diese Wirkstoffe nicht als solche, sondern als Wirkstoffformulierungen oral verabreicht. Die Wirkstoffe können beispielsweise in Tabletten, Kapseln, Säften oder Pellets enthalten sein. Die orale Einnahme von Medikamenten stellt auch für Wirkstoffe mit bitterem Geschmack generell eine bevorzugte Art der Verabreichung dar.

Es ist bekannt, dass ältere Patienten, Kinder und Patienten mit Erkrankungen bzw. Verletzungen im Bereich des Ösophagus mitunter Probleme haben, größere Darreichungsformen, wie z.B. Oblongtabletten oder Kapseln, herunterzuschlucken. Für diese Patienten wurden bestimmte Applikationsmittel entwickelt, um ihnen die Einnahme oraler Darreichungsformen zu erleichtern. So sind spezielle Trinkhalme bekannt, in denen eine wirkstoffhaltige Pelletformulierung enthalten ist. Der Patient nimmt dann die Pelletformulierung zu sich, indem er den Trinkhalm wie einen herkömmlichen Strohhalm zum Trinken einer geeigneten Flüssigkeit benutzt. Die Pellets werden dann über den Flüssigkeitsstrom transportiert. In diesem Zusammenhang kann beispielsweise vollumfänglich verwiesen werden auf WO 2003/079957, WO 2004/000202 und WO2004/000264.

Die Formulierung von Wirkstoffen mit bitterem Geschmack in herkömmlichen Pellets verhindert jedoch nicht, dass der bittere Geschmack des enthaltenen Wirkstoffs vom Patienten wahrgenommen werden kann. Viele herkömmliche Formulierungen verhindern die Entfaltung des bitteren Geschmacks des enthaltenen Wirkstoffs nicht oder nur unzureichend.

Dies hängt damit zusammen, dass die Pellets vor dem Herunterschlucken für ein Zeitintervall im Mundraum - und im Falle der Einnahme mit Hilfe eines Trinkhalms in der Transportflüssigkeit - verweilen, welches ausreicht, dass der Bitterstoff ggf. über den Speichel als Transportmedium mit den Geschmackspapillen der Zunge in Kontakt kommt. Der bittere Geschmack des Wirkstoffs wird dann entweder während oder unmittelbar nach der oralen Einnahme vom Patienten wahrgenommen.

In solchen Fällen entwickelt der Patient mitunter eine ausgeprägte Aversion gegen die Einnahme der Formulierung, was u.a. der strikten Einhaltung eines bestimmten Therapieschemas abträglich sein kann. Auch kann durch den bitteren Geschmack ein Würgereflex ausgelöst werden, der eine effektive orale Verabreichung verhindert oder zumindest erschwert. Dieses Problem tritt insbesondere bei Kindern und älteren Patienten auf.

Es gibt im Stand der Technik verschiedene Ansätze, den bitteren Geschmack von Wirkstoffen zu maskieren.

Beispielsweise ist der Zusatz von herkömmlichen Süß- oder Aromastoffen zum Maskieren des unangenehmen Geschmacks allgemein bekannt. Diese Süß- oder Geschmacksstoffe verleihen der Formulierung einen Eigengeschmack, der den bitteren Geschmack des Wirkstoffs überdecken soll. Durch eine solche Art der Geschmacksmaskierung gelingt es in manchen Fällen, den Geschmack von mäßig bitteren pharmazeutischen Wirkstoffen zu maskieren. Bei extrem bitteren Wirkstoffen ist diese Art der Geschmacksmaskierung jedoch unzureichend.

Ferner ist es im Stand der Technik bekannt, Pellets zur Maskierung des bitteren Geschmacks des enthaltenen Wirkstoffs mit Überzügen zu versehen (siehe z.B. WO 2006 (069920). Die Materalien, aus denen diese Filmüberzüge hergestellt sind, umfassen üblicherweise in Wasser unlösliche oder schwerlösliche Polymere, welche durch den Speichel nicht oder zumindest nicht vollständig abgelöst werden, so dass der wirkstoffhaltige Kern durch den Filmüberzug geschützt und dadurch der bittere Geschmack des enthaltenen Wirkstoffs für die Dauer der Einnahme maskiert wird.

Derartige Filmüberzüge haben jedoch den Nachteil, dass sie auch einen erheblichen Einfluss auf das Freisetzungsverhalten haben. So führt die Unlöslichkeit bzw. Schwerlöslichkeit des Filmüberzugs in wässrigen Medien dazu, dass sich der Filmüberzug im Magen nicht oder nur sehr langsam ablöst, so dass es häufig zu einer Verzögerung oder sogar einer Retardierung der Wirkstofffreisetzung kommt. Diese Verzögerung bzw. Retardierung kann durchaus vorteilhaft und gewünscht sein, je nachdem, um welche Art von Wirkstoff es sich handelt und für welche medizinische Indikation die Formulierung vorgesehen ist. So können im Sauren unlösliche Polymere beispielsweise als magensaftresistente Beschichtungsmaterialien eingesetzt werden, um eine Freisetzung des Wirkstoffs erst im Darmmilieu zu ermöglichen. Beispiele für magensaftresistente Beschichtungsmaterialien sind Eudragit^{®} L-55, Eudragit^{®} L, Eudragit^{®} S oder Eudragit^{®} FS bzw. Cellulosederivate wie z.B. HP55, HPMCAS oder CAP.

Die Retardierungswirkung des Filmüberzugs und der physiologische Ort der Freisetzung können dadurch gesteuert werden, dass dem unlöslichen Polymer wasserlösliche Substanzen als Porenbildner beigemengt werden. Wird ein derartiger Filmüberzug einem wässrigen Medium ausgesetzt, so lösen sich die Porenbildner aus dem Filmüberzug heraus und lassen wassergefüllte Poren zurück, durch die der Wirkstoff entweichen kann. Über Größe und Anzahl der Poren kann die Freisetzung des Wirkstoffs reguliert werden.

Üblicherweise dauert das Herauslösen der wasserlöslichen Porenbildner so lange, dass die Verweilzeit im Mund - und im Falle der Einnahme mit Hilfe eines Trinkhalms in der Transportflüssigkeit - nicht ausreicht, um bereits Wirkstoff aus der Formulierung freizusetzen. Eine wirksame Maskierung des bitteren Geschmacks des Wirkstoffs kann daher auf diese Weise realisiert werden. Ein Beispiel für einen solchen Filmüberzug ist Eudragit^{®} L-55 mit Saccharose oder Zitronensäure als wasserlösliche Porenbildner.

Für Darreichungsformen, welche den Wirkstoff rasch, ohne Retardierung freisetzen sollen (*rapid release* oder *immediate release*), sind solche Filmüberzüge häufig ungeeignet, da die Bildung der Poren infolge des Herauslösens des wasserlöslichen Porenbildners eine gewisse Zeit in Anspruch nimmt. Eine Beschleunigung der Wirkstofffreisetzung kann allenfalls durch Erhöhen der Menge an wasserlöslichem Porenbildner erreicht werden.

So zeigen Experimente mit Pellets, welche mit einem Überzug aus Eudragit^{®} L-55 und einer ausreichenden Menge an Zitronensäure versehen sind, dass einerseits der bittere Geschmack des Wirkstoffs effektiv maskiert werden kann und dass andererseits unter *in vitro* Bedingungen eine Freisetzungsrate des Wirkstoffs erreicht wird, welche mit der von nicht überzogenen Pellets vergleichbar ist (*immediate release*).

Ein Nachteil dieser herkömmlichen Filmüberzüge besteht jedoch darin, dass *in vivo* die Bioverfügbarkeit des Wirkstoffs in mit diesen Filmüberzügen versehenen Darreichungsformen unter Fed-Status-Test Bedingungen nicht der Bioverfübarkeit herkömmlicher Darreichungsformen, insbesondere solcher mit sehr schneller Freisetzung (*rapid release*, *immediate release*. z.B. Saftformulierungen oder zerfallende Pellets ohne Überzug) entspricht, d.h. es besteht keine Bioäquivalenz.

*Fed-Status-Test* Bedingungen bei Bioäquivalenzuntersuchungen sind z.B. immer dann erforderlich, wenn Bioäquivalenz für Arzneistoffe bzw. Arzneimittel gezeigt werden soll, die gemäß Beipackzettel zusammen mit Nahrung eingenommen werden sollen oder bei Einnahme mit Nahrung eine im Vergleich zum *Fasted Status* veränderte Bioverfügbarkeit zeigen. Dies wird auch in den behördlichen Zulassungsrichtlinien vorgeschrieben. In diesem Zusammenhang kann beispielsweise vollumfänglich verwiesen werden auf *Guidance for Industry, U.S. Department of Health and Human Services, FDA, Center for Drug Evaluation and Research (CDER): Bioavailability and Bioequivalence Studies for Orally Administered Drug Products* und *Food-Effect Bioavailability and Fed Bioequivalence Studies;* und *GUIDANCE FOR INDUSTRY, Bioequivalence Requirements: Comparative Bioavailability Studies Conducted in the Fed State, Health Canada, file number:* 05-114865-164, 2005/06/08*.*

Es ist bekannt, dass die Verabreichung eines Wirkstoffs mit Nahrung *(Fed Status)* im Vergleich zur Verabreichung des Wirkstoffs ohne Nahrung *(Fasted Status)* eine Verringerung, Verzögerung oder Erhöhung der Wirkstoffabsorption, aber auch überhaupt keinen Einfluss auf die Wirkstoffabsorption haben kann (vgl. z.B. N. Yasui-Furukori et al., J Clin Pharmacol, 55, 2003, 382-8). Durch die Nahrungsaufnahme ändern sich die Bedingungen im Gastointestinaltrakt grundlegend, insbesondere im Hinblick auf Motilität, pH-Wert, Ionenkonzentration, Pufferkapazität, Osmolarität, Flüssigkeitsvolumen und Konzentration an oberflächenaktiven Substanzen (Gallensäurenkonzentration).

Der Einfluss der Nahrung auf die Pharmakokinetik von Cefpodoxim proxetil bei oraler Verabreichung als Suspension wird beispielsweise erläutert von G.S. Hughes et al., Clin Pharmacol Ther 1989, 46, 674-85; G.L. Keams et al., Pediatr Infect Dis J., 1998, 17(9), 799-804; und M.T. Borin et al., Antimicrob Agents Chemother, 1995, 273-5. Der Einfluss der Nahrung auf die Pharmakokinetik von Cefuroxim axetil bei oraler Verabreichung bzw. intravenöser Verabreichung wird beispielsweise erläutert von A Finn et al., Biopharm Drug Dispos. 1987, 8(6), 519-26. Auch Azithromycin wird in der Praxis bevorzugt unter *Fed Status* Bedingungen verabreicht (vgl. z.B. G.W. Amsden et al., J Antimicrob Chemother 2001, 47, 61-6).

Der Erfindung liegt die Aufgabe zugrunde, Darreichungsformen zur Verfügung zu stellen, welche Vorteile gegenüber den Darreichungsformen des Standes der Technik aufweisen. Die Darreichungsformen sollten nach Möglichkeit insbesondere für schwerlösliche Wirkstoffe eine unverzögerte Wirkstofffreisetzung aufweisen (*rapid release* oder *immediate release*) und dabei insbesondere auch unter Fed-Status-Test Bedingungen bioäquivalent zu einer entsprechenden Saftformulierung sein. Ferner sollten die Darreichungsformen auch von Patienten mit Schluckbeschwerden eingenommen werden können.

Es wurde überraschend gefunden, dass bei Beschichtung von Pellets mit bestimmten Filmüberzügen folgende Eigenschaften verwirklicht werden können:
(i) eine wirksame Maskierung des Geschmacks des in den Pellets enthaltenen Wirkstoffs,
(ii) eine rasche Freisetzung *(rapid release* oder *immediate release)* auch für schwerlösliche Wirkstoffe,
(iii) ein Freisetzungsprofil des Wirkstoffs unter *in vitro* Bedingungen, welches mit dem Freisetzungsprofil unbeschichteter Pellets vergleichbar ist, und
(iv) *in vivo* Bioäquivalenz der Pellets zu einer Saftformulierung des Wirkstoffs selbst unter Fed-Status-Test Bedingungen.

Gegenstand der Erfindung sind beschichtete Pellets, welche einen in Wasser schwerlöslichen pharmazeutischen Wirkstoff enthalten, unter *in vitro* Bedingungen in Phosphat-Puffer bei pH 5,0 nach 30 Minuten zumindest 80% des Wirkstoffs freisetzen und
mit einer Zusammensetzung beschichtet sind, die ein Wachs (A) und einen Hydrogelbildner (B) umfasst, wobei das Wachs (A)
(i) einen HLB-Wert ≤ 5, und/oder
(ii) einen Schmelzbereich ≥ 60°C, und/oder
(iii) einen Erstarrungsbereich Δ von weniger als 35°C, und/oder
(iv) eine Dichte ≥ 0,80 g cm⁻³
aufweist,
wobei der schwerlösliche Wirkstoff bei 23°C in reinem Wasser eine Löslichkeit von höchstens 20 mg ml⁻¹ aufweist und ein Antibiotikum ist, welches
- gegen gram⁺ und/oder gegen gram⁻ Bakterien und/oder
- bakteriostatisch, bakterizid und/oder bakteriolytisch
wirksam ist.

Ein Gegenstand der Erfindung betrifft beschichtete Pellets wie beansprucht, welche
- unter *in vitro* Bedingungen in Phosphat-Puffer bei pH 5,0 (vorzugsweise auch bei pH 6,4 oder pH 6,8) nach 30 Minuten zumindest 80%, bevorzugt zumindest 85%, bevorzugter zumindest 90%, noch bevorzugter zumindest 92,5%, am bevorzugtesten zumindest 95% und insbesondere zumindest 97,5% des Wirkstoffs freisetzen und
- unter *in vivo Fasted-* und/oder Fed-Status-Test-Bedingungen gemäß den geltenden Bioäquivalenzkriterien für den jeweiligen Wirkstoff bioäquivalent zu einer Saftformulierung des Wirkstoffs sind.

Es wurde überraschend gefunden, dass beschichtete Pellets mit rascher Wirkstofffreisetzung, welche einen schwerlöslichen pharmazeutischen Wirkstoff enthalten, hergestellt werden können, die sowohl
a) unter *in vitro* Bedingungen ein im wesentlichen unverzögertes Freisetzungsverhalten zeigen, welches unbeschichteten Pellets vergleichbar ist, als auch
b) selbst unter *in vivo Fed-Status-Test* Bedingungen bioäquivalent zu einer Saftformulierung des Wirkstoffs sind.

Mit herkömmlichen beschichteten Pellets (z.B. Beschichtung aus Eudragit^{®} L-55 + Zitronensäure) konnte bisher nur Voraussetzung a) erfüllt werden; Bioäquivalenz mit einer Saftformulierung war hingegen unter Fed-Status-Test Bedingungen (Voraussetzung b) nicht gegeben.
Figur 1 zeigt das Freisetzungsprofil von unbeschichteten Cefpodoxim proxetil Pellets bei pH 5,0 (ohne SLS) (2 Chargen mit Standardabweichung für Mittelwert aus drei parallelen Tests) (vgl. Vergleichsbeispiel 5).
Figur 2 zeigt den Vergleich der Freisetzung von mit 10% Carnaubawachs/Carbomer beschichteten Cefpodoxim Pellets (A, Beispiel 1) und unbeschichteten Cefpodoxim-Pellets (B, Vergleichsbeispiel 5) in Phosphat-Puffer bei pH 5,0 ohne SLS (mit Standardabweichung für Mittelwert aus drei parallelen Tests).
Figur 3a zeigt den Vergleich der Freisetzung von unbeschichteten Cefpodoxim Pellets (A, Vergleichsbeispiel 5) zu mit Camaubawachs/Carbomer-beschichteten Cefpodoxim Pellets (B, Beispiel 1) und mit Eudragit/Zitronensäure-beschichteten Cefpodoxim Pellets (C, Vergleichsbeispiel 6) bei pH 5,0 in Phosphat-Puffer + 0.1 % SLS. Figur 3b zeigt den analogen Vergleich (A,B,C) bei pH 6,4 in Phosphat-Puffer ohne SLS. Figur 3c zeigt den analogen Vergleich (A,B,C) bei pH 5,0 in Phosphat-Puffer ohne SLS.
Figur 4 zeigt die Freisetzung von Cefpodoxim Pellets beschichtet mit Carnaubawachs und Xanthangummi (Hydrogelbildner (B)) bei verschiedenen Überzugsmengen (in Gew.-%) bei pH 5,0 in Phosphatpuffer (Beispiel 2, A 20% Auftrag, B 15% Auftrag, C 10% Auftrag, D 5% Auftrag).
Figur 5 zeigt die Freisetzung von Cefpodoxim Pellets beschichtet mit Carnaubawachs und verschiedenen Hydrogelbildnern (B) bei pH 5,0 in Phosphatpuffer (Beispiel 3, A unbeschichtete Cefpodoxim-Pellets; B 10 Gew.-% Camaubawachs/L-HPC; C 10 Gew.-% Carnaubawachs/Guargummi; D 10 Gew.-% Carnaubawachs/ Xanthangummi; E 9 Gew.-% Carnaubawachs/Carbomer).
Figur 6 zeigt die Bioverfügbarkeit erfindungsgemäßer beschichteter Pellets (A, Beispiel 1) verglichen mit einer äquimolaren Referenzsuspension (B, Orelox^{®}) jeweils bei einer Dosis von 100 mg Cefpodoxim unter Fed-Status-Testbesingungen.

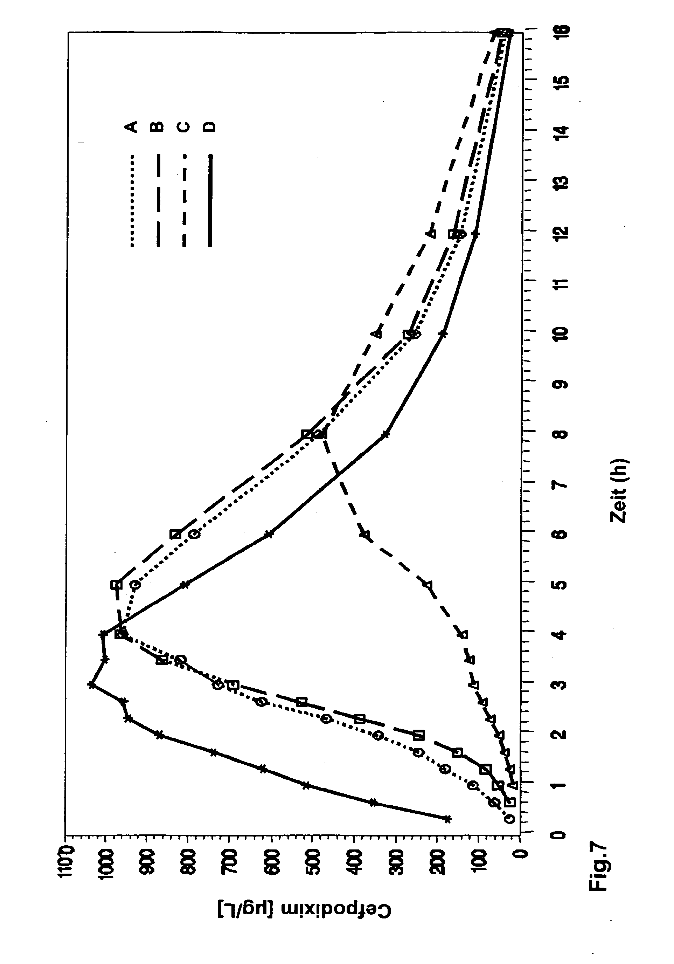

Figur 7 zeigt die Bioverfügbarkeit unbeschichteter Cefpodoxim Pellets (A, Vergleichsbeispiel 5), erfindungsgemäß mit Camaubawachs/Carbomer beschichteter Cefpodoxim Pellets (B, Beispiel 1), mit Eudragit/Zitronensäure beschichteter Cefpodoxim Pellets (C, Vergleichsbeispiel 6) und einer (Orelox®-)Saftformulierung (D), jeweils bei einer Dosis von 80 mg Cefpodoxim (Beispiel 6).
Figur 8 zeigt die Ergebnisse potentiometrischer Untersuchungen *(electronic tongue)* zur Wirksamkeit der Beschichtung der erfindungsgemäßen Pellets im Hinblick auf die Maskierung des bitteren Geschmacks von Cefpodoxim proxetil.

Bei den erfindungsgemäßen Pellets handelt es sich um Formulierungen mit einer im wesentlichen nicht retardierten Wirkstofffreisetzung. Dies kommt dadurch zum Ausdruck, dass unter *in vitro* Bedingungen z.B. in Phosphat-Puffer bei pH 5,0 (vorzugsweise auch bei pH 6,4 oder pH 6,8) nach 30 Minuten zumindest 80% des Wirkstoffs freigesetzt werden. Geeignete Methoden zur Bestimmung des Freisetzungsprofils sind dem Fachmann bekannt und umfassen die Blattrührer- bzw. die Drehkörbchenmethode. Bezüglich weiterer Einzelheiten kann beispielsweise auf die einschlägigen Arzneibücher verwiesen werden, z.B. USP oder Ph. Eur.

Die erfindungsgemäß beschichteten Pellets weisen einen Kern auf, welcher von einer Beschichtung umgeben ist. Vorzugsweise bedeckt die Beschichtung vollständig die Oberfläche des Kerns. In einer bevorzugten Ausführungsform weisen die erfindungsgemäßen Pellets einen wirkstoffhaltigen Kern und eine wirkstofffreie, äußere Beschichtung auf. Der Aufbau und die Herstellung von beschichteten Pellets ist dem Fachmann bekannt (vgl. z.B. Bauer et al., "Lehrbuch der pharmazeutischen Technologie" 6. Auflage, WVG Stuttgart, 1999).

Besonders bevorzugt liegen die erfindungsgemäßen Pellets in Form von beschichteten Extrusionspellets vor, vorzugsweise in Form von beschichteten, sphäroiden Extrusionspellets.

Es ist jedoch grundsätzlich auch möglich, dass die Pellets als Aufbaupellets vorliegen. Aufbaupellets können beispielsweise einen wirkstofffreien Kern, z.B. in Form von Zucker- oder Maisstärkepellets, aufweisen, welcher von einer wirkstoffhaltigen Beschichtung umgeben ist, die ihrerseits eine äußere, wirkstofffreie Beschichtung aufweist. Der Kern kann seinerseits aus mehreren Schichten radial aufgebaut sein, wobei sowohl der Kern als auch die einzelnen Schicht(en) jeweils unabhängig voneinander einen oder mehrere Wirkstoff(e) und/oder einen oder mehrere Hilfsstoff(e) enthalten können.

Die erfindungsgemäßen Pellets weisen vorzugsweise einen mittleren Durchmesser im Bereich von 100 bis 1000 µm, bevorzugter 150 bis 900 µm, noch bevorzugter 180 bis 850 µm, am bevorzugtesten 200 bis 800 µm und insbesondere 250 bis 710 µm auf.

Die erfindungsgemäßen Pellets sind vorzugsweise auch unter *in vivo Fed-Status-*Test-Bedingungen bioäquivalent zu einer Saftformulierung. Durch den *in-vivo Fed-Status* Test wird die Bioverfügbarkeit von Formulierungen pharmazeutischer Wirkstoffe, welche zusammen mit der Nahrung aufgenommen werden müssen oder bei gemeinsamer Gabe mit Nahrung eine veränderte Bioverfügbarkeit aufweisen, *in vivo* festgestellt. Dieser Test wird unter anderem zur Ermittlung der Bioäquivalenz von pharmazeutischen Formulierungen herangezogen (bevorzugt sind die erfindungsgemäßen Pellets zusätzlich auch unter *in vivo* Fasted-Status-Test-Bedingungen bioäquivalent zu einer Saftformulierung).

Die Bioverfügbarkeit ist eine pharmakologische Messgröße für den Anteil eines Wirkstoffs, der unverändert im systemischen Kreislauf zur Verfügung steht. Sie ist ein Maß dafür, wie schnell und in welchem Umfang der Wirkstoff resorbiert wird und am Wirkort zur Verfügung steht.

Die Bioäquivalenz ist die Austauschbarkeit zweier Darreichungsformen bzw. Formulierungen, welche den gleichen Wirkstoff enthalten. Diese werden dann als bioäquivalent angesehen, wenn sie eine vergleichbare Bioverfügbarkeit aufweisen.

Pharmakokinetische Parameter, an Hand derer die Bioäquivalenz in den meisten Fällen beurteilt werden kann, sind die Fläche unter der Plasmaspiegel-Zeit Kurve (AUC_{0-inf}) und der maximale Plasmaspiegel (Cₘₐₓ).

Damit Bioäquivalenz vorliegt, muss das 90%-Konfidenzintervall des Quotienten der für die zu vergleichenden Kenngrößen ermittelten durchschnittlichen Werte innerhalb eines Akzeptanzintervalls liegen, welches üblicherweise von 80% bis 125% reicht. Diese Kriterien bzw. Definitionen für Bioäquivalenz sind dem Fachmann allgemein bekannt. In diesem Zusammenhang kann beispielsweise auf die Europäische Richtlinie zur Ermittlung von Bioäquivalenz CPMP/EWP/QWP/1401/98 vom 26. Juli 2001, Seite 10, Punkt 3.6.2 verwiesen werden. Erfindungsgemäß bevorzugt sind Bioverfügbarkeit und Bioäquivalenz definiert wie in 21 CFR 320, 314.50(d)(3) und 314.94(a)(7).

Die erfindungsgemäßen Pellets sind bioäquivalent zu einer Saftformulierung. Die Saftformulierung, welche in diesem Zusammenhang als Referenz dient, enthält vorzugsweise die identische Menge an Wirkstoff. Bevorzugt hat die Saftformulierung die Zusammensetzung einer vorzugsweise im Jahre 2006 marktgeführten Saftformulierung des jeweiligen schwerlöslichen Wirkstoffs. Ist keine Saftformulierung kommerziell verfügbar, so hat die Saftformulierung vorzugsweise folgende Zusammensetzung: 5 ml der zubereiteten Suspension bzw. Lösung enthalten: empfohlene Einzeldosis des jeweiligen Wirkstoffs; Carmellose-Calcium, Natriumchlorid, Natrium-L-hydrogenglutamat, Aspartam, Eisenoxidhydrat (E 172), Carmellose-Na, Saccharose (5ml enth. 0,05 BE), Citronensäure ·1 H₂O, Hyprolose, Sorbitantrioleat, Talkum, hochdisperses Siliciumdioxid, Bananenaroma, Kaliumsorbat, Lactose ·1H₂O.

Ist der schwerlösliche Wirkstoff Cefpodoximproxetil, so handelt es sich bei der Saftformulierung bevorzugt um Orelox^{®} pediatrique aus Frankreich bzw. Orelox^{®} junior Deutschland in der im Jahre 2006 marktgeführten Zusammensetzung, besonders bevorzugt um Orelox^{®} junior 40 mg aus Deutschland (Sankyo. München), z.B. Charge # 01 E758.

Ein weiterer Gegenstand der Erfindung betrifft beschichtete Pellets, welche
- einen in Wasser schwerlöslichen pharmazeutischen Wirkstoff enthalten,
- unter *in vitro* Bedingungen in Phosphat-Puffer bei pH 5,0 (vorzugsweise auch bei pH 6,4 oder pH 6,8) nach 30 Minuten zumindest 80%, bevorzugt zumindest 85%, bevorzugter zumindest 90%, noch bevorzugter zumindest 92,5%, am bevorzugtesten zumindest 95% und insbesondere zumindest 97,5% des Wirkstoffs freisetzen und
- mit einer Zusammensetzung beschichtet sind, die eine lipophile Komponente (A) und einen Hydrogelbildner (B) umfasst,
wobei die reine lipophile Komponente (A)

Das Wachs (A) weist
(i) einen HLB -Wert ≤ 5,0, bevorzugter ≤ 4,0, noch bevorzugter ≤ 3,5, am bevorzugtesten ≤ 3,0, und insbesondere ≤ 2,5, und/oder
(ii) einen Schmelzbereich ≥ 60°C, bevorzugt ≥ 65°C, bevorzugter ≥ 70°C, noch bevorzugter ≥ 75°C, am bevorzugtesten ≥ 80°C, und insbesondere ≥ 82°C, und/oder
(iii) einen Erstarrungsbereich Δ (Temperatur, bei der vollständig geschmolzen, bis Temperatur, bei der Schmelze erstarrt) von ≤ 35°C, bevorzugt ≤ 30°C, bevorzugter ≤ 25°C, noch bevorzugter ≤ 20°C, am bevorzugtesten ≤ 15°C, und insbesondere ≤ 10°C, und/oder
(iv) eine Dichte ≤ 0,85 g cm⁻³, bevorzugt ≤ 0,90 g cm⁻³, bevorzugter ≥ 0,92 g cm⁻³, noch bevorzugter ≥ 0,94 g cm⁻³, am bevorzugtesten ≥ 0,96 g cm⁻³ und insbesondere ≥ 0,98 g cm⁻³, und bevorzugt
(v) einen Brechungsindex n_{D}⁹⁰ ≥ 1,2000, bevorzugt ≥ 1,3000, bevorzugter ≥ 1,3500, noch bevorzugter ≥ 1,4000, am bevorzugtesten ≥ 1,4250 und insbesondere ≥ 1,4400
auf.

Der HLB-Wert wird vorzugsweise mit der Methode nach Griffin et al. bestimmt (W. C. Griffin, J. Soc. Cosmet. Chem. 1, 1949). Schmelz- und Erstarrungsbereich, Dichte und Brechungsindex werden vorzugsweise gemäß Arzneibuch bzw. ASTM bestimmt. Die Bestimmung des Schmelzpunkts bzw. Schmelzbereichs erfolgt vorzugsweise gemäß der Kapillarmethode (EurPh 2.2.14) bzw. durch Thermoanalyse (EurPh 2.2.34).

Die lipophile Komponente (A) ist ein Wachs. Wachse gehören zur Stoffklasse der Lipide. Es handelt sich um Ester von Fettsäuren mit langkettigen aliphatischen Alkoholen. Wachse im Sinne der Erfindung können natürlich (tierischen oder pflanzlichen Ursprungs), semi-synthetisch oder synthetisch sein.

Die Verseifungszahl des Wachses liegt bevorzugt im Bereich von 70 bis 97, bevorzugter 72 bis 95, noch bevorzugter 74 bis 93, am bevorzugtesten 76 bis 91 und insbesondere 78 bis 89. Besonders bevorzugt handelt es sich bei dem Wachs (A) um ein Wachs ausgewählt aus der Gruppe bestehend aus Walrat, Schellackwachs, Cetylstearylalkohol, Cetylstearylpalmitat, Zuckerester, Hartfett, mikrokristallines Wachs, Bienenwachs und Carnaubawachs, wobei Carnaubawachs besonders bevorzugt ist. Carnaubawachs ist ein natürliches Wachs, das aus den Blättern der Carnaubapalme *(Copernica cerifera)* gewonnen wird.

Die Verwendung von Carnaubawachs als Beschichtungs- oder Matrixmaterial ist im Stand der Technik bekannt. Herkömmliche Formulierungen, welche mit Carnaubawachs beschichtet sind, weisen jedoch aufgrund der Wasserunlöslichkeit und des hohen Schmelzbereichs dieser Substanz eine retardierte Freisetzung auf, was für die erfindungsgemäßen Pellets gerade unerwünscht ist.

Als Hydrogelbildner (B) können synthetische, natürliche und/oder semi-synthetische Polymere verwendet werden, die in Wasser quellbar sind und generell hydrophile Eigenschaften haben.

Vorzugsweise weist der Hydrogelbildner (B) eine Temperaturbeständigkeit bis mindestens 505°C, bevorzugter mindestens 75°C, noch bevorzugter mindestens 100°C, am bevorzugtesten mindestens 125°C und insbesondere mindestens 150°C auf. Temperaturbeständigkeit bedeutet in diesem Zusammenhang, dass bei Erwärmung des Hydrogelbildners (B) auf die jeweilige Temperatur für 10 Minuten und Abkühlen auf Raumtemperatur keine signifikanten Eigenschaftsänderungen eintreten im Vergleich zu einer Referenzprobe des Hydrogelbildners (B), welche nicht erwärmt wurde.

Vorzugsweise weist der Hydrogelbildner (B) bei Raumtemperatur und 80% rel. Feuchtigkeit eine Feuchtigkeitsaufnahme von mindestens 20 Gew.-%, bevorzugter mindestens 30 Gew.-%, noch bevorzugter mindestens 40 Gew.%, am bevorzugtesten mindestens 50 Gew.-% und insbesondere mindestens 60 Gew.-% auf. Bestimmt wird die Feuchtigkeitsaufnahme mittels *Dynamic Vapor Sorption* (DVS). Bevorzugte Hydrogelbildner (B) sind synthetische bzw. semi-synthetische Polymere, wie Cellulosen und deren Derivate, wie beispielsweise Hydroxyethylcellulose, Hydroxypropylcellulose, insbesondere niedrig-substituierte Hydroxypropylcellulose (L-HPC), Na-Carboxymethylcellulose, Methylethylcellulose, etc. "L-HPC" ist dem Fachmann bekannt. "Niedrig substituiert" bedeutet üblicherweise, dass der Gewichtsanteil von Hydroxypropoxy-Substituenten an den Cellulosegerüstmolekülen im Bereich von 5-16 Gew.-% liegt.

Vorzugsweise ist der Hydrogelbildner (B) ausgewählt aus der Gruppe, bestehend aus Alginsäure, Alginat, amidiertem Pektin, Propylenglycolalginat, Carbomer, Dammar-Gummi, Dextrinen, Furcellaran, Guargummi, Guarkernmehl, Gellan, Ghatti-Gummi, Gummi arabicum, Gummi aus Fichtensaft, Johannisbrotkernmehl, Karayagummi, Keratin, Konjakmehl, L-HPC, Locust Bean Gum, Mastix, Pektin, Tarakernmehl, Traganth, Chitosan und Xanthangummi.

Ferner ist es bevorzugt, dass der Hydrogelbildner (B) ausgewählt ist aus der Gruppe, bestehend aus den Substanzen E 400, E 401, E 402, E 403, E 404, E 405, E 406, E 407, E 408, E 409, E 410, E 411, E 412, E 413, E 414, E 415, E 416, E 417, E 418, E 419, E 420, E 421, E 422, E 423, E 424, E 425, E 426, E 427, E 428, E 429, E 430, E 431, E 432, E 433, E 434, E 435, E 436, E 437, E 438, E 439, E 440, E 441, E 442, E 443, E 444, E 445, E 446, E 447, E 448, E 449, E 450, E 451, E 452, E 453, E 454, E 455, E 456, E 457, E 458, E 459, E 460, E 461, E 462, E 463, E 464, E 465, E 466, E 467, E 468, E 469, E 470, E 471, E 472, E 473, E 474, E 475, E 476, E 477, E 478, E 479, E 480, E 481, E 482, E 483, E 484, E 485, E 486, E 487, E 488, E 489, E 490, - E 491, E 492, E 493, E 494, E 495, E 496, E 497, E 498, E 499, E 1404, E 1410, E 1412, E 1413, E 1414, E 1420, E 1422, E 1440, E 1442, E 1450 und E 1451. Die europäischen Zusatzstoffkennzeichnungsnummern ("E-Nummern") zur Kennzeichnung von Zusatzstoffen in Lebensmitteln werden gemäß den Richtlinien der Europäischen Behörde für Lebensmittelsicherheit (EBL) klassifiziert und sind dem Fachmann bekannt. In diesem Zusammenhang wird auf den *"*General Standard for Food Additives" (Joint FAO/WHO Expert Committee on Food Additives (JECFA), vorzugsweise Stand Juni 2005, verwiesen, auf dem die europäischen Zusatzstoffkennzeichnungsnummem basieren.

Bevorzugt weist der Hydrogelbildner (B) einen mittleren Partikeldurchmesser d₅₀ im Bereich von 2,5 bis 150 µm auf, bevorzugter 5,0 bis 25 µm oder 80 bis 140 µm, noch bevorzugter 7,5 bis 22,5 µm oder 90 bis 130 µm, am bevorzugtesten 10 bis 20 µm oder 100 bis 120 µm und insbesondere 12,5 bis 17,5 µm oder 105 bis 115 µm.

Bei den erfindungsgemäßen Pellets beträgt die Schichtdicke des Überzugs, dargestellt als Gewichtszunahme (g/g) der Kerns durch Aufbringen des Überzugs, bevorzugt 1-50%, bevorzugter 2-30%, noch bevorzugter 5-20% und insbesondere 10-15%.

Der Gewichtsanteil des Hydrogelbildners (B) am Gesamtgewicht des Filmüberzugs liegt bevorzugt im Bereich von 2,5 bis 50 Gew.-%, bevorzugter von 5,0 bis 40 Gew.-%, noch bevorzugter von 7,5 bis 30 Gew.-%, am bevorzugtesten von 10 bis 20 Gew.-% und insbesondere von 12,5 bis 17,5 Gew.-%.

Es scheint, dass durch den Hydrogelbildner (B) in dem Wachs (A) überraschenderweise eine schnelle Wirkstofffreisetzung ermöglicht wird, da der Hydrogelbildner bei Kontakt mit Wasser aufquillt und dadurch die lipophile Komponente aufgrund ihrer vergleichsweise hohen Sprödbrüchigkeit vom Pelletkem abplatzt. Dies sollte jedoch nicht als Bindung an eine wissenschaftliche Theorie aufgefasst werden.

Neben dem Wachs (A) und dem Hydrogelbildner (B) kann die Zusammensetzung, mit der die erfindungsgemäßen Pellets beschichtet sind, übliche Hilfsstoffe enthalten, z.B. Füllmittel, Weichmacher, Gleitmittel, Farbstoffe, Aromen und/oder Konservierungsstoffe. Derartige Hilfsstoffe sind dem Fachmann bekannt. Diesbezüglich kann beispielsweise vollumfänglich verwiesen werden auf H.P. Fiedler, Lexikon der Hilfsstoffe für Pharmazie, Kosmetik und angrenzende Gebiete, Editio Cantor Aulendorf, 2001. Bevorzugt enthält die Zusammensetzung jedoch neben dem Wachs (A) und dem Hydrogelbildner (B) keine weiteren Bestandteile.

Die erfindungsgemäßen Pellets zeichnen sich durch eine hervorragende Lagerstabilität aus. Viele lipophile Substanzen, wie z.B. Kakaobutter oder Precirol^{®}, sind polymorph und haben deshalb die generelle Eigenschaft, bei Lagerung in Abhängigkeit von den Lagerungsbedingungen mit der Zeit die innere Struktur zu verändern. Verwendet man diese Substanzen als Beschichtungsmaterialien für Pellets, so geht mit der Modifikationsänderung infolge der Lagerung üblicherweise eine Veränderung des Freisetzungsverhaltens einher. Das Freisetzungsprofil der Pellets ist dann nicht lagerstabil.

Es wurde überraschend gefunden, dass bestimmte lipophile Substanzen mit vergleichsweise hohem Schmelzbereich bei Lagerung keine solche Modifikationsänderung und damit ein lagerstabiles Freisetzungsprofil zeigen.

Die erfindungsgemäßen Pellets weisen bevorzugt bei Lagerung ein im wesentlichen unverändertes Freisetzungsverhalten auf. Vorzugsweise weisen die erfindungsgemäßen Pellets vor und nach Lagerung für 1 Monat bei 30°C und 60% RF, bevorzugter bei 35°C und 70% RF, noch bevorzugter bei 40°C und 75% RF, im wesentlichen ein unverändertes Freisetzungsprofil auf. Bevorzugter ist das Freisetzungsverhalten der erfindungsgemäßen Pellets nach Lagerung für 2 Monate, noch bevorzugter für 3 Monate, am bevorzugtesten für 4 Monate und insbesondere für 6 Monate unter den vorstehend genannten Bedingungen im wesentlichen unverändert. "Im wesentlichen unverändert" bedeutet in diesem Zusammenhang, dass zu jedem Zeitpunkt während der Messung der freigesetzten Wirkstoffmenge *in vitro* der Messwert nach der Lagerung bevorzugt um höchstens 20%, bevorzugter um höchstens 15%, am bevorzugtesten um höchstens 10% und insbesondere um höchstens 5% von dem entsprechenden Messwert vor der Lagerung abweicht.

Ein in Wasser schwerlöslicher pharmazeutischer Wirkstoff im Sinne der Beschreibung ist ein Wirkstoff mit einer Löslichkeit von bevorzugt höchstens 20 mg ml⁻¹, bevorzugter höchstens 10 mg ml⁻¹, noch bevorzugter höchstens 5 mg ml⁻¹, am bevorzugtesten höchstens 1 mg ml⁻¹ und insbesondere höchstens 0,5 mg ml⁻¹ bei 23°C in reinem Wasser.

Die erfindungsgemäßen Pellets enthalten einen Wirkstoff, für den die Bioäquivalenz nicht zwangsläufig gemäß den einschlägigen Richtlinien unter Fed-Status-Test Bedingungen untersucht werden muss. So betrifft die Erfindung auch Pellets, welche einen Wirkstoff enthalten, für den die Bioäquivalenz gemäß den einschlägigen Richtlinien unter Fasted-Status-Test Bedingungen untersucht wird. Die Eigenschaft der erfindungsgemäßen Pellets, dass sie unter *in vivo* Fed-Status-Test Bedingungen bioäquivalent zu einer Saftformulierung des Wirkstoffs sind, ist somit nicht einschränkend im Hinblick auf die Natur des Wirkstoffs zu verstehen, da auch solche Wirkstoffe umfasst sind, für welche die Bioäquivalenzuntersuchungen üblicherweise nicht unter Fed-Status-Test Bedingungen, sondern unter Fasted-Status-Test Bedingungen durchgeführt werden.

Bevorzugt enthalten die erfindungsgemäßen Pellets einen Wirkstoff mit bitterem Geschmack. Objektive Methoden zur Bestimmung des Geschmackes von Stoffen sind dem Fachmann bekannt. Beispielsweise ist dies mit Hilfe potentiometrischer Verfahren möglich, z.B. mit sog. "elektronischen Zungen" *(electronic tongue,* vgl. Vaslov et al., Pure Appl. Chem., 2005, 77(11), 1965-83). Ansonsten kann auch ein relativer Vergleich gegenüber einem Placebo mittels einer Testpanelbeurteilung erfolgen.

Die erfindungsgemäßen Pellets enthalten als Wirkstoff wenigstens ein Antibiotikum. Antibiotika im Sinne der Erfindung sind gegen gram⁺ und/oder gegen gram⁻ Bakterien und/oder bakteriostatisch, bakterizid und/oder bakteriolytisch wirksam. Besonders bevorzugt sind die Antibiotika bakteriostatisch und/oder bakteriolytisch wirksam. Antibiotika können über verschiedene Mechanismen ihre Wirksamkeit entfalten. In einer bevorzugten Ausführungsform ist das Antibiotikum ein Zellwandsynthesehemmer und/oder ein Hemmer der Proteinbiosynthese am Ribosom, und/oder ein Gyrase-Hemmer, und/oder ein Folsäureantagonist und/oder ein Hemmer der bakteriellen RNA-Polymerase.

Je nach Aktivität bzw. Struktur werden die Antibiotika in verschiedene Gruppen eingeteilt. Vorzugsweise ist das Antibiotikum ausgewählt aus der Gruppe der Tetracycline [ATCJ01A], Amphenicole [ATCJ01B], β-Lactam-Antibiotika, Penicilline [ATCJ01C], anderen β-Lactam-Antibiotika [ATCJ01 D], Sulfonamide und Trimethoprim [ATCJ01 E], Macrolide, Lincosamide und Streptogramine [ATCJ01 F], Aminoglycosid-Antibiotika [ATCJ01G], Chinolone [ATCJ01M] oder anderen Antibiotika [ATCJ01 X]. Die in eckigen Klammern angegebenen Bezeichnungen entsprechen dabei dem ATC-Index, wie er von der WHO zur Klassifizierung der Arzneistoffe verwendet wird (bevorzugter Stand: Januar 2005 oder 2006). Hinsichtlich weiterer Einzelheiten zum ATC-Index kann beispielsweise verwiesen werden auf U. Fricke, *"Anatomisch-therapeutisch-chemische Klassifikation mit Tagesdosen: Amtliche Fassung des A TC-Index mit DDD-Angaben für Deutschland im Jahre 2006",* Wissenschaftliches Institut der AOK oder unter "www.gelbe-liste.de".

Bevorzugt handelt es sich bei dem Wirkstoff um ein Cephalosporin, besonders bevorzugt um ein Cephalosporin ausgewählt aus der Gruppe bestehend aus Cefaclor, Cefacetril, Cefadroxil, Cefalexin, Cefaloglycin, Cefaloridin, Cefalosporin C, Cefalotin, Cefamandol, Cefapirin, Cefatrizin, Cefazedon, Cefazolin, Cefdinir, Cefepim, Cefetamet, Cefixim, Cefmenoxim, Cefmetazol, Cefodizim, Cefonicid, Cefoperazon, Ceforanid, Cefotaxim, Cefotetan, Cefotiam, Cefoxitin, Cefpimizol, Cefpiramid, Cefpirom, Cefpodoxim, Cefprozil, Cefradin, Cefroxadin, Cefsulodin, Ceftazidim, Cefteram, Ceftezol, Ceftibuten, Ceftizoxim, Ceftriaxon, Cefuroxim, Cefuzonam, Latamoxef, Loracerbef und Pivcefalexin, oder deren pharmazeutisch verträglichen Estern, wie z.B. Cefpodoximproxetil oder Cefuroximaxetil; um ein Cephamycin ausgewählt aus der Gruppe bestehend aus Cefbuperazon, Cefmetazol, Cefminox, Cefotetan und Cefoxitin; oder um ein Makrolidantibiotikum ausgewählt aus der Gruppe bestehend aus Azithromycin, Carbomycin, Clarithromycin, Erythromycin, Josamycin, Leucomycin, Midecamycin, Miokamycin, Oleandomycin, Primycin, Rokitamycin, Rosaramicin, Roxithromycin, Spiramycin und Troleandomycin, oder deren pharmazeutisch verträglichen Estern, wie z.B. Erythromycinacistrat, Erythromycinestolat, Erythromycinglucoheptonat, Erythromycinlactobionat, Erythromycinpropionat oder Erythromycinstearat.

In einer bevorzugteren Ausführungsform ist das Antibiotikum ausgewählt aus der Gruppe der Cephalosporine der 2. Generation [ATCJ01 DC], Cephalosporine der 3. Generation [ATCJ01DD] oder Makrolide [ATCJ01 FA], noch bevorzugter ist das Antibiotikum ausgewählt aus der Gruppe bestehend aus Azithromycin [J01 FA1 0], Cefpodoxim [J01 DD13], Cefpodoximproxetil, Cefuroxim [J01 DC02], Cefuroximaxetil [J01 DC13], Cefixim [J01 DD08], Cefdinir [J01 DD15] und Clarithromycin [J01 FA09].

Die erfindungsgemäßen Pellets weisen neben der Beschichtung einen Kern auf. Der Kern ist vorzugsweise wirkstoffhaltig. Neben dem Wirkstoff kann der Kern der erfindungsgemäßen Pellets übliche Hilfsstoffe enthalten, z.B. Füllmittel, Bindemittel, Weichmacher, Sprengmittel, Rundungsmittel, Gleitmittel, Farbstoffe, Aromen und/oder Konservierungsstoffe.

In einer bevorzugten Ausführungsform enthält der Kern der erfindungsgemäßen Pellets einen Zuckerester.

Zuckerester sind Mono-, Di-, Tri-, Oligo- oder Polyester von Zuckern mit Säuren. Als Zucker kommen Mono-, Di-, Oligo- und/oder Polysaccharide in Frage, wie beispielsweise Erythrose, Threose, Xylose, Lyxose, Ribose, Allose, Altrose, Glucose, Mannose, Gulose, Idose, Galaktose, Talose, Psicose, Fructose, Sorbose, Tagatose, Fucose, Sucrose (Saccharose), Lactose, Maltose, Trehalose, Cellobiose etc.

Als Säure kommen einerseits Carbonsäuren, insbesondere Fettsäuren, wie z.B. Palmitinsäure, Stearinsäure, Ölsäure, Linolsäure, Linolensäure, Arachidonsäure, etc., andererseits anorganische Oxosäuren, wie z.B. Schwefelsäure oder Phosphorsäure, in Frage. Zuckerester der Schwefelsäure können auch als sulfatisierte Saccharide bezeichnet werden.

Als Ester von Carbonsäuren sind die Mono-, Di- oder Triester von gesättigten Fettsäuren mit Mono- oder Disacchariden erfindungsgemäß bevorzugte Zuckerester. Besonders bevorzugt sind Mono-, Di- und Tripalmitate der Saccharose (Sucrose), bzw. deren Mischungen.

Als Ester von anorganische Oxosäuren sind sulfatisierte Polysaccharide erfindungsgemäß bevorzugte Zuckerester, insbesondere Carrageenan, z.B. 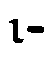 , λ- und/oder κ-Carrageenan, wobei κ-Carrageenan besonders bevorzugt ist.

Bevorzugt enthält der Kern der erfindungsgemäßen Pellets sowohl einen Fettsäureester der Saccharose, als auch ein Carrageenan.

Bevorzugt enthalten die erfindungsgemäßen Pellets im Kern Ca₃(PO₄)₂.

Besonders bevorzugte Ausführungsformen 1 bis 5 der erfindungsgemäßen Pellets sind in nachfolgender Tabelle zusammengefasst:

| | 1 | 2 | 3 | 4 | 5 |
|---|---|---|---|---|---|
| Beschichtung | | | | | |
| lipophile Komponente (A) | Schmelzbereich ≥ 60°C | Schmelzbereich ≥ 80°C | Wachs mit Schmelzbereich ≥ 80°C | Camaubawachs | Carnaubawachs |
| Hydrogelbildner (B) | Polymer mit Gewichtszunahme ≥ 20% bei RT/80% RF | Polymer mit Gewichtszunahme ≥ 40% bei RT/80% RF | Xanthangummi, Guargummi, Carbomer oder L-HPC | Xanthangummi, Guargummi, Carbomer oder L-HPC | Xanthangummi, Guargummi, Carbomer oder L-HPC |
| Kern | | | | | |
| Wirkstoff | Antibiotikum | [ATCJ01DC], [ATCJ01DD] oder [ATCJ01FA] | [ATCJ01DC], [ATCJ01DD] oder [ATCJ01FA] | [ATCJ01DC], [ATCJ01DD] oder [ATCJ01FA] | Azithromycin, Cefuroximaxetil, Cefixim, Cefdinir, oder Cefpodoximproxetil |
| Hilfsstoff(e) | Zuckerester | Zuckerester | sulfatisiertes Polysaccharid | Carrageenan | κ-Carrageenan + Zuckerester mit HLB > 12 |

Die erfindungsgemäßen Pellets werden vorzugsweise hergestellt, indem man die Ausgangsstoffe mischt, granuliert, extrudiert und ggf. formt, vorzugsweise sphäronisiert.

Vorzugsweise liegen die erfindungsgemäßen Pellets als Extrusionspellets vor und können nach folgendem Verfahren hergestellt werden:
1) Einwiegen des Wirkstoffs und der Inhaltsstoffe des Pelletkerns;
2) Mischen;
3) Feuchtgranulieren unter Zusatz von gereinigtem Wasser;
4) Extrudieren;
5) Sphäronisieren;
6) Trocknen;
7) Klassieren;
8) Einwiegen der Komponenten (A) und (B);
9) Schmelzen der Komponente (A);
10) Dispergieren der Komponente (B) in der geschmolzenen Komponente (A);
11) Einwiegen der Pellets aus Schritt 9);
12) Erwärmen der Pellets auf 40-60°C;
13) Beschichten der Pellets mit der in Schritt 12) erhaltenen Dispersion;
14) Abkühlen der beschichteten Pellets auf 40°C; und
15) Klassieren.

Dem Fachmann ist bekannt, dass die Komponenten gleichzeitig oder nacheinander der Mischung zugesetzt werden können. Ebenfalls kann die Mischung in einem bekannten Mischer oder Granulierer erfolgen, so dass ggf. Mischung, Granulierung und Extrusion in einem Gerät erfolgen können. Die Granulierung kann durch Feuchtgranulierung, vorzugsweise mit Wasser oder wässrigen Lösungen mit gelöstem Bindemittel erfolgen. Dem Fachmann sind geeignete wässrige Lösungen bekannt (z.B. PVP, HPMC Lösung zum Granulieren).

Die Sphäronisation, Extrusion und das Überziehen können jeweils in den dem Fachmann bekannten Apparaturen erfolgen. Für das Überziehen kann eine Wirbelschicht-Apparatur eingesetzt werden.

Ein weiterer Aspekt der vorliegenden Erfindung betrifft eine orale Darreichungsform umfassend die vorstehend beschriebenen Pellets.

In einer bevorzugten Ausführungsform der erfindungsgemäßen Pellets bzw. der erfindungsgemäßen Darreichungsform wird nach oraler Verabreichung die maximale Plasmakonzentration Cₘₐₓ des Wirkstoffs nach tₘₐₓ im Bereich von 1 h bis 8 h, bevorzugter von 1,5 h bis 7 h, noch bevorzugter von 2 h bis 6 h, am bevorzugtesten von 2,5 bis 5,5 h und insbesondere von 3 h bis 5 h erreicht.

Ein weiterer Aspekt der Erfindung betrifft die Verwendung eines vorstehend beschriebenen Wirkstoffs zur Herstellung von vorstehend beschriebenen Pellets oder zur Herstellung einer vorstehend beschriebenen Darreichungsform zur Vorbeugung und/oder Behandlung von bakteriellen Erkrankungen.

Vorzugsweise werden die erfindungsgemäßen Pellets bzw. Darreichungsformen zur Vorbeugung/und oder Behandlung von bakteriellen Erkrankungen verwendet, welche durch Bakterien hervorgerufen werden umfassend *Staphylococcus sp., Streptococcus sp., Escherichia coli, Klebsiella sp, Enterobacter sp., Citrobacter sp., Providencia sp., Haemophilus sp., Peptostreptococcus sp., Pseudomonas sp., Acinetobacter sp., Proteus sp., Serratia marcescens, Proteus vulgaris, Proteus mirabilis, Proteus penneri, Shigella sp., Salmonella sp., Clostridium sp., Mycobacterium sp., Listeria sp., Meningococcus sp., Candidas sp., Nocardia sp.* und/oder *Treponema sp.*

Bevorzugt werden die erfindungsgemäßen Pellets bzw. Darreichungsformen zur Vorbeugung/und oder Behandlung von bakteriellen Erkrankungen verwendet ausgewählt aus der Gruppe bestehend aus Hautinfektionen, Wundinfektionen, Infektionen der Weichteile, Infektionen der Harnwege, Genitalinfektionen, Brustinfektionen, Ohrinfektionen, Infektionen der Atemwege, Naseninfektionen, Nasennebenhöhlenentzündungen, Halsinfektionen und Entzündungen im Rachenbereich.

Bevorzugter ist die bakterielle Erkrankung ausgewählt aus der Gruppe bestehend aus Haut- und Wundinfektion, Infektionen der Weichteile, unkomplizierter Infektionen der Harnwege, Genitalinfektionen, bestimmter durch Clamydien bedingter Infektionen der Harnwege und Geschlechtsorgane, akuter Gonokokkeninfektion der Frau, Entzündungen im Rachenbereich, Ohrinfektionen, Naseninfektionen, Nasennebenhöhlenentzündungen, Infektionen der Atemwege einschließlich Lungenentzündungen, Brustinfektionen und Halsinfektionen; insbesondere akuter Bronchitis, akuter und akut exazerbierter chronischer Bronchitis, superinfizierter Bronchitis, akuter Verschlimmerung der chronischen Bronchitis, akustischer Neuritis, akuter gonorrhoischer Urethritis des Mannes, akuter Otitis media, Angina, Aortitis, Arthritis, bakterieller Pneumonie, Bronchopneumonie, Bursitis, Candidiasis, Cervicitis, Cholera, Chorioenteritis, Conjunktivitis, Cystitis, Diphterie, Encephalitis, Endocarditis, Enteritis, Enterocolitis, Enterohaemorrhagie, Epididymitis, Episcleritis, Laryngitis, Lepra, Leptospirosis, Leukoderma, Listeriosis, Lymphogranuloma inguinale, Mittelohrentzündung, Morbus Lyme, Myocarditis, Myositis, Neuritis, Nocardiosis, Orchitis, Osteomyelitis, Pericarditis, Periostitis, Peritonitis, Pharyngitis, Prostatitis, Pyelonephritis, Rickettsiosis, Salmonellosis, Sepsis, Shigellosis, Sinusitis, Synovitis, Syphilis, Tetanus, Typhus, Tonsillitis, Trichomoniasis, Tuberkulose, tuberkulöser Meningitis, Urethritis und Vulvovaginitis.

Bevorzugte Patientengruppen sind pädiatrische (bis einschließlich 14 Jahre alt) und/oder geriatrische Patienten (ab 60 Jahre alt).

Die erfindungsgemäßen Pellets können vorzugsweise als Einmaldosis in einem Applikationssystem umfassend einen Trinkhalm mit vorzugsweise beweglicher Sperrvorrichtung, wie sie in WO 2003/079957, WO 2004/000202, WO2004/000264 beschrieben ist, angeordnet sein. Als Transportflüssigkeiten eignen sich partikelfreie Getränke, vorzugsweise wässrige Flüssigkeiten, wie z.B. Wasser, vorzugsweise Mineralwasser, Tee, Fruchtsäfte, Coca-Cola und/oder Limonaden, wobei säuerliche Getränke besonders bevorzugt sind.

Die Erfindung wird durch die nachfolgenden Beispiele näher erläutert. Diese Beispiele sind jedoch nicht einschränkend auszulegen.

### Vergleichsbeispiel 1: (unbeschichtete Clarithromycin Pellets)

Extrusionspellets mit folgender Zusammensetzung

| pro Dosis | Ausgangsstoffe |
|---|---|
| 250,0 mg | Clarithromycin Ph. Eur. |
| 100,0 mg | κ-Carrageenan |
| 50,0 mg | Tricalciumphosphat Ph. Eur. |
| 20,0 mg | Saccharoseester S-1570, E473 |

wurden durch Mischung der Ausgangsstoffe in einem Schnellmischer und anschließender Feuchtgranulation und Extrusion des feuchten Granulats durch einen Extruder mit einer 0,5 x 0,5 mm Extrusionsmatrize hergestellt. In einem geeigneten Sphäroniser wurden die Extrudate sphäronisiert und die so erhaltenen Pellets in einem Wirbelschichttrockner bis zu einer Restfeuchte unter 10% getrocknet. Die getrockneten Pellets wurden mit Hilfe der Siebmethode klassiert und die 250 bis 710 µm Fraktion aller Siebungen vereinigt.

Von diesen nicht überzogenen Pellets wurde zunächst die Freisetzung mit einer Freisetzungsapparatur mit Blattrührer gemäß U.S. Pharmacopeia in 900 ml

Phosphat-Pufferlösung (pH 6,4) bei 37°C des Freisetzungsmediums und einer Umdrehungsgeschwindigkeit von 100 min⁻¹ für 60 Minuten gemessen. Die jeweils zu einem Zeitpunkt freigesetzte Menge des Wirkstoffs wurde durch HPLC oder UVphotometrisch bestimmt.

Die nachstehenden Werte zeigen das entsprechende Freisetzungsprofil als Mittelwert von 3 parallelen Bestimmungen:

| Clarithromycin | | | | | |
|---|---|---|---|---|---|
| Minuten | 0,0 | 15,0 | 30,0 | 45,0 | 60,0 |
| % | 0 | 58 | 90 | 102 | 106 |

Somit wurden bereits nach 30 Minuten mehr als 80% des Wirkstoffs freigesetzt.

### Vergleichsbeispiel 2: (Unbeschichtete Cefixim Pellets)

Analog Vergleichsbeispiel 1 wurden Extrusionspellets mit folgender Zusammensetzung hergestellt:

| pro Dosis | Ausgangsstoffe |
|---|---|
| 447,6 mg | Cefixim x 3H₂O, mikronisiert entsprechend |
| 400,0 mg | Cefixim USP |
| 50,6 mg | Tricalciumphosphat Ph. Eur. |
| 194,8 mg | Carrageenan NF |

Die Freisetzung des Wirkstoffs aus den nicht überzogenen Pellets wurde nach der in Beispiel 1 angegebenen Methode in 900 ml Pufferlösung bei einem pH-Wert 6,8 als Mittelwert von 3 parallelen Bestimmungen ermittelt und nachstehend aufgeführt:

| Cefixim | | | | | |
|---|---|---|---|---|---|
| Minuten | 0,0 | 15,0 | 30,0 | 45,0 | 60,0 |
| % | 0 | 87 | 94 | 94 | 94 |

Somit wurden bereits nach 30 Minuten mehr als 80% des Wirkstoffs freigesetzt.

### Vergleichsbeispiel 3: (unbeschichtete Amoxicillin Pellets)

Analog Vergleichsbeispiel 1 wurden Extrusionspellets mit folgender Zusammensetzung hergestellt:

| pro Dosis | Ausgangsstoffe |
|---|---|
| 575,00 mg | Amoxicillin-Trihydrat Ph. Eur., entsprechend |
| 500,00 mg | Amoxicillin, wasserfrei |
| 65,00 mg | Tricalciumphosphat, Ph. Eur. |
| 250,00 mg | κ-Carrageenan |

Von den vereinigten Siebfraktionen mit einer Teilchengröße von 250 bis 710 µm wurde die Freisetzung des Wirkstoffs aus den nicht überzogenen Pellets in Phosphat-Puffer (pH 6,8) in 900 ml für 30 Minuten nach der in Beispiel 1 angegebenen Methode als Mittelwert von 3 parallelen Bestimmungen ermittelt und in der nachfolgenden Tabelle angegeben:

| Amoxicillin | | | | | | |
|---|---|---|---|---|---|---|
| Minuten | 0,0 | 1,0 | 5,0 | 10,0 | 15,0 | 30,0 |
| % | 0 | 41 | 101 | 100 | 98 | 96 |

Somit wurden bereits nach 30 Minuten mehr als 80% des Wirkstoffs freigesetzt.

### Vergleichsbeispiel 4: (Unbeschichtete Azithromycin Pellets)

Analog zu Vergleichsbeispiel 1 wurden Extrusionspellets mit folgender Zusammensetzung hergestellt:

| pro Dosis | Ausgangsstoffe |
|---|---|
| 20,0 mg | Saccharoseester S-1570 |
| 256,0 mg | Azithromycin (Monohydrat) entsprechend |
| 250,0 mg | Azithromycin wasserfrei |
| 50,0 mg | Tricalciumphosphat, Ph. Eur. |
| 100,0 mg | κ-Carrageenan, |

### Vergleichsbeispiel 5: (unbeschichtete Cefpodoxim 80 mg Pellets)

Herstellung von 2 kg Cefpodoxim Pelletkernen und Abfüllung in einen Trinkhalm

### Zusammensetzung:

| pro Dosis | Ausgangsstoffe |
|---|---|
| 104,35 mg | Cefpodoxim proxetil entsprechend |
| 80,00 mg | Cefpodoxim |
| 20,88 mg | Tricalciumphosphat |
| 41,76 mg | κ-Carrageenan |
| 8,36 mg | Saccharoseester S-1570 |

Die Ausgangsstoffe wurden vermischt, feuchtgranuliert und anschließend das feuchte Granulat durch einen Extruder mit einer 0,5 x 0,5 mm Extrusionsmatrize hergestellt. In einem geeigneten Sphäroniser wurden die Extrudate sphäronisiert und die so erhaltenen Pellets in einem Wirbelschichttrockner bis zu einer Restfeuchte unter 10% getrocknet. Die getrockneten Pellets wurden mit Hilfe der Siebmethode klassiert, in Trinkhalme abgefüllt und in Alufolie eingesiegelt.

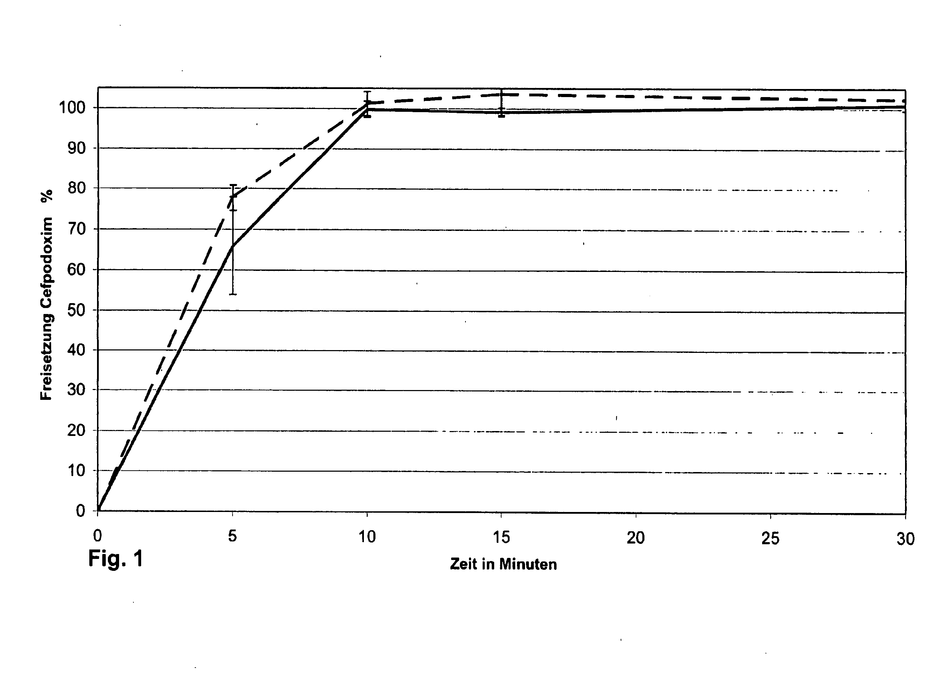

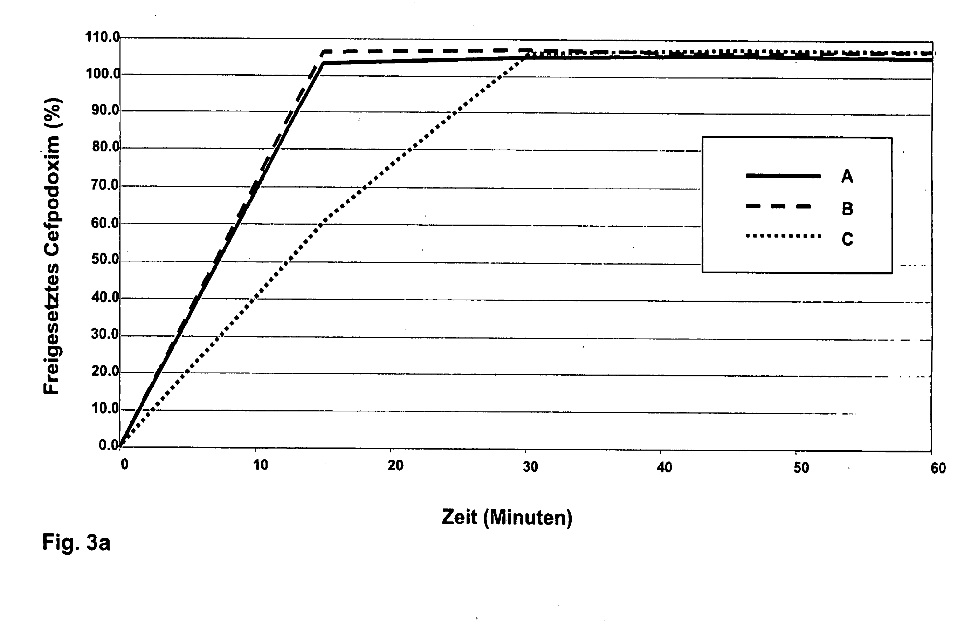

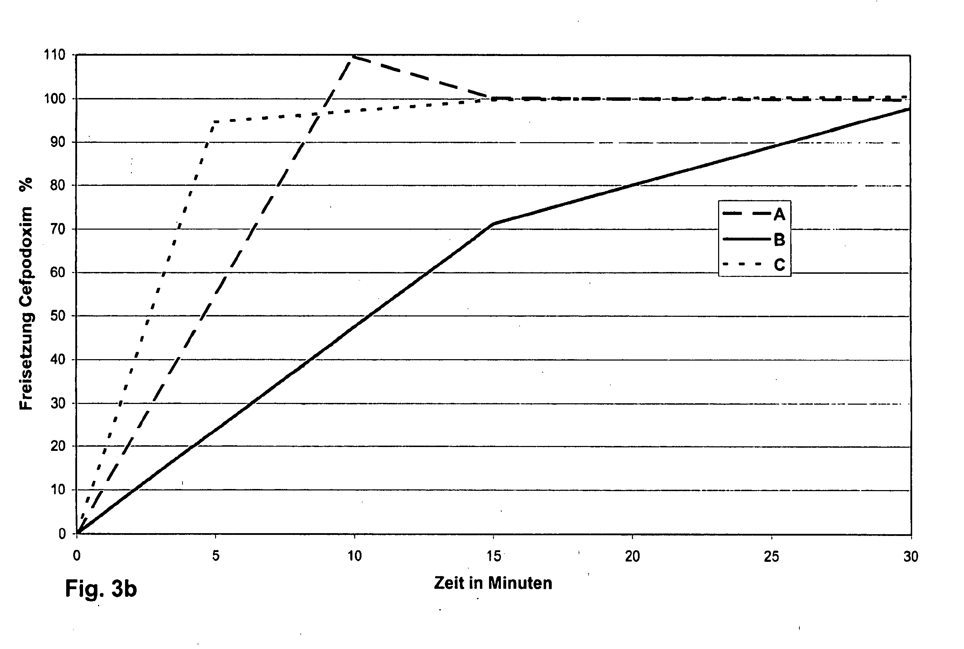

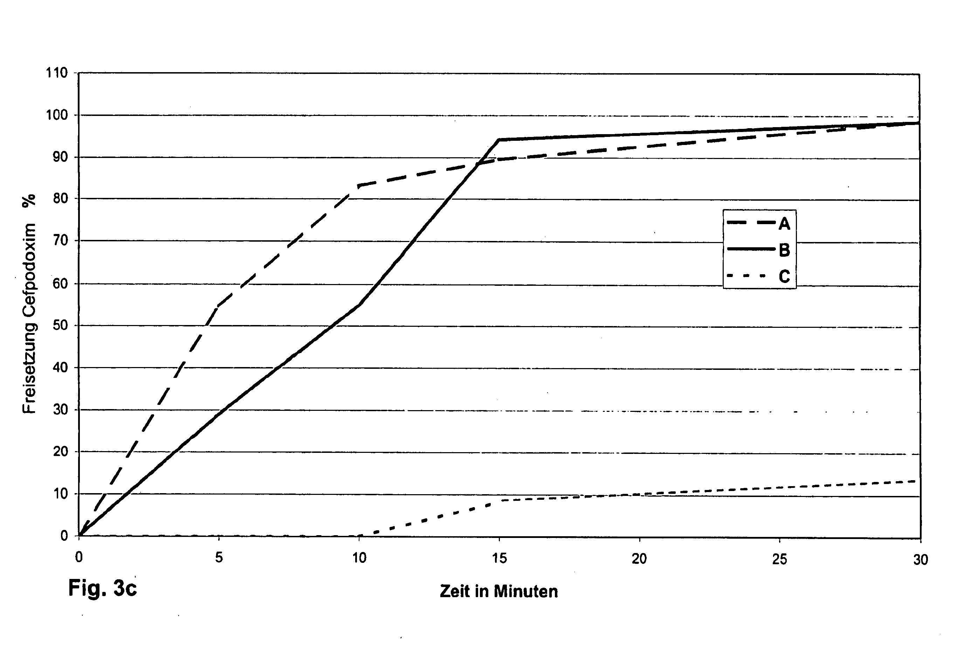

Die Freisetzung des Wirkstoffs aus den nicht überzogenen Pellets wurde nach der in Vergleichsbeispiel 1 angegebenen Methode jeweils bei 100 U min⁻¹ in 1000 ml Phosphat-Puffer, pH 5,0 *(fed status, simulated intestinal fluid)* (Figuren 1, 3a und 3c) bzw. 1000 ml Phosphat-Puffer pH 6,4 (Figur 3b) *(fasted state, simulated intestinal fluid*) bestimmt.

Die Ergebnisse der Freisetzung von Cefpodoxim sind in Figuren 1 und 3a-c abgebildet. Bei den unbeschichteten Pellets werden nach 30 Minuten in allen Medien jeweils mehr als 80% des Cefpodoxims abgegeben. Die Anwesenheit eines lösungsverbessemden Tensids (SLS) ist nicht erforderlich, da die Pellets zerfallen und der Wirkstoff nicht über löslichkeitsabhängige Diffusion freigesetzt wird.

### Vergleichsbeispiel 6: (Cefpodoxim Pellets - beschichtet mit Eudragit L-55 30D + Zitronensäure)

Herstellung von 4 kg Cefpodoxim Pelletkernen und Abfüllung in einen Trinkhalm

### Zusammensetzung:

| | |
|---|---|
| 104,350 mg | Cefpodoxim proxetil entsprechend |
| 80,000 mg | Cefpodoxim |
| 20,880 mg | Tricalciumphosphat |
| 41,760 mg | κ-Carrageenan |
| 8,360 mg | Zuckerester S-1570 |
| | |
| 29,187 mg | L-55 30D |
| 1,530 mg | Eudragit Zitronensäure |
| | |
| 3,589 mg | Triethylcitrat |
| 0,041 mg | Polysorbat 80 |
| 1,354 mg | Glycerolmonostearat |

Die Herstellung der Pelletkerne erfolgte analog Vergleichsbeispiel 5. Die Pelletkerne wurden mit 18 Gew.-% Taste Masking Coat (Eudragit L55 30D + Zitronensäure) und anschließend mit 2 Gew.-% Overcoat (Eudragit L55 30D ohne Zitronensäure) beschichtet. Die beschichteten Pellets wurden klassiert und in Trinkhalme abgefüllt.

### Beispiel 1: (Cefpodoxim Pellets - beschichtet mit Carnaubawachs + Carbomer)

Herstellung von 4 kg Cefpodoxim Pelletkernen, Überziehen mit 10 Gew.-% Carnaubawachs/Carbomer und Abfüllung in Trinkhalm

### Zusammensetzung:

| | |
|---|---|
| 104,35 mg | Cefpodoxim proxetil entsprechend |
| 80,00 mg | Cefpodoxim |
| 20,88 mg | Tricalciumphosphat |
| 41,76 mg | κ-Carrageenan |
| 8,36 mg | Zuckerester S-1570 |
| | |
| 14,90 mg | Carnaubawachs |
| 2,63 mg | Carbomer |

Die Herstellung der Pelletkerne erfolgte analog Vergleichsbeispiel 5. Anschließend wurden die Pelletkerne mit einem Überzug aus Carnaubawachs und Carbomer beschichtet und auf einen Durchmesser im Bereich von 250-710 µm klassiert. Danach wurden die beschichteten Pellets in Trinkhalme abgefüllt und in Alufolie eingesiegelt.

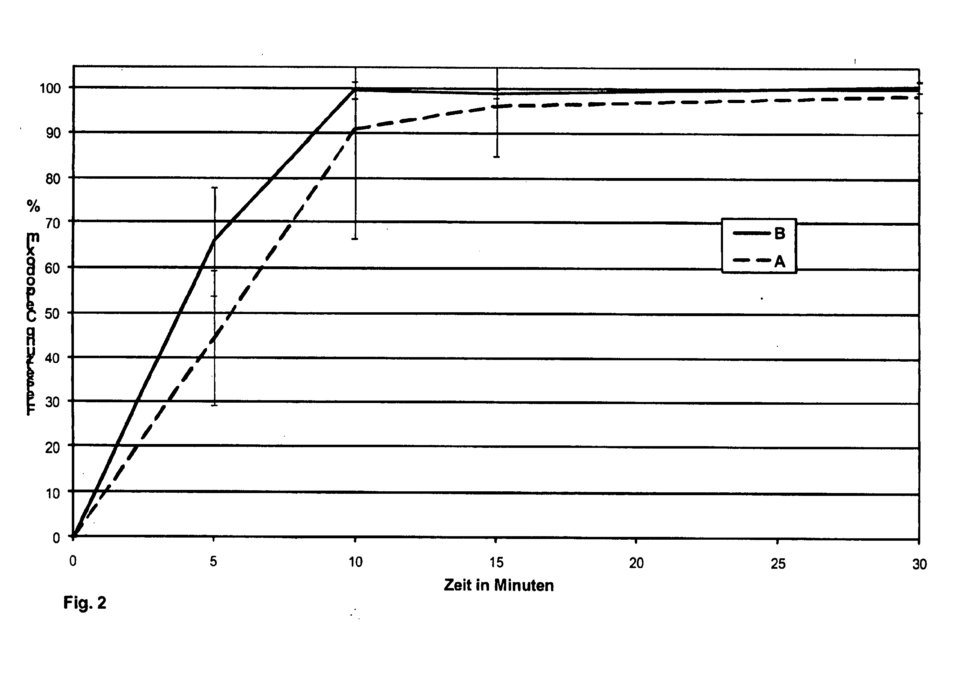

Die Freisetzung des Wirkstoffs aus den beschichteten Pellets wurde nach der in Vergleichsbeispiel 1 angegebenen Methode jeweils in 900 bzw. 1000 ml Na⁺-Phosphat-Puffer, pH 5,0 mit und ohne SDS und in Phosphatpuffer pH 6,4 bei 100 U min⁻¹ über eine Dauer von jeweils 30 bzw. 60 Minuten bestimmt. Das Ergebnis zur Freisetzung von Cefpodoxim ist in Figuren 2 und 3a, b abgebildet.

Aus Figur 2 ist ersichtlich, dass das Freisetzungsprofil von Cefpodoxim bei unbeschichteten Pellets (A, Vergleichsbeispiel 5) und das Freisetzungsprofil von Cefpodoxim bei mit Camaubawachs/Carbomer beschichteten Pellets (B, Beispiel 1) in Phosphat-Puffer bei pH 5,0 im wesentlichen gleich ist.

Aus Figuren 3a-c ist ersichtlich, dass die erfindungsgemäßen Carnaubawachs/ Carbomer-beschichteten Pellets (B, Beispiel 1) Cefpodoxim praktisch gleich schnell wie unbeschichtete Pellets (A, Vergleichsbeispiel 5) freisetzen. Die mit Eudragit/ Zitronensäure überzogenen Pellets (C, Vergleichsbeispiel 6) weisen eine langsamere Freisetzungskinetik auf. Bei allen drei Pelletformulierungen werden jedoch nach 30 Minuten in Na⁺-Phosphat-Puffer bei pH 5,0 + 0,1% SLS und bei pH 6,4 mehr als 80% des Cefpodoxims freigesetzt (Figur 3a).

Nur die erfindungsgemäßen Pellets setzen - ebenso wie die nicht überzogenen Pellets - auch schon bei pH 5,0 ohne SDS >80% des Wirkstoffs in 20 Minuten frei. Die verdeutlicht, dass trotz des Zusatzes der Porenbildner die Freisetzung aus den mit Eudragit L-55/Zitronensäure überzogenen Pellets für den schwerlöslichen Wirkstoff zu langsam ist, um bei pH 5,0 ohne SLS eine nennenswerte Freisetzung zu erzielen. Erst bei pH 6,4, bei dem das Polymer Eudragit L-55 aufgelöst wird, erfolgt die Freisetzung ähnlich schnell wie aus unbeschichteten Pellets.

Insbesondere nach Nahrungsaufnahme entspricht jedoch der pH-Wert des Dünndarms eher dem Wert von 5,0 (Dressmann et al.) und ermöglicht somit keine Auflösung des an pH 5,5 löslichen Polymers. Die wassergefüllten Poren allein erlauben dem schwerlöslichen Cefpodoxim proxetil jedoch nur in Gegenwart des Solubilisators SLS im Dissolutionsmedium eine ausreichende Austrittsgeschwindigkeit.

Die erfindungsgemäßen Pellets zeigen jedoch unabhängig von den Milieubedingungen in allen Fällen ein spontanes "Aufplatzen" des Überzugs und somit analoge, schnelle Freisetzung wie die nicht überzogenen Pellets.

### Beispiel 2: (Cefpodoxim Pellets - beschichtet mit Carnaubawachs + Xanthangummi in unterschiedlichen Mengen)

Herstellung von Cefpodoxim Pelletkernen, Überziehen mit Carnaubawachs/ Xanthangummi und Abfüllung in Trinkhalm

### Zusammensetzung:

| Kerne | | | | | |
|---|---|---|---|---|---|
| | Cefpodoxim proxetil entsprechend | 104,35 mg | | | |
| | Cefpodoxim | 80,00 mg | | | |
| | Tricalciumphosphat | 20,88 mg | | | |
| | κ-Carrageenan | 41,76 mg | | | |
| | Zuckerester S-1570 | 8,36 mg | | | |
| | Überzug | 5% | 10% | 15% | 20% |
| | Carnaubawachs | 10,85 | 21,70 | 32,55 | 43,40 |
| | Xanthangummi | 1,92 | 3,84 | 5,76 | 7,68 |

Die Cefpodoxim-Granulate wurden analog zum Verfahren nach Beispiel 1 hergestellt.

Die Pelletkerne wurden mit 5, 10, 15 und 20 Gew.-% des Carnaubawachs/Xanthangummi-Überzugs bei einer Einlass-Temperatur von 60-100°C bei einer Sprührate von 0,6 bis 1,8 ml/min. beschichtet.

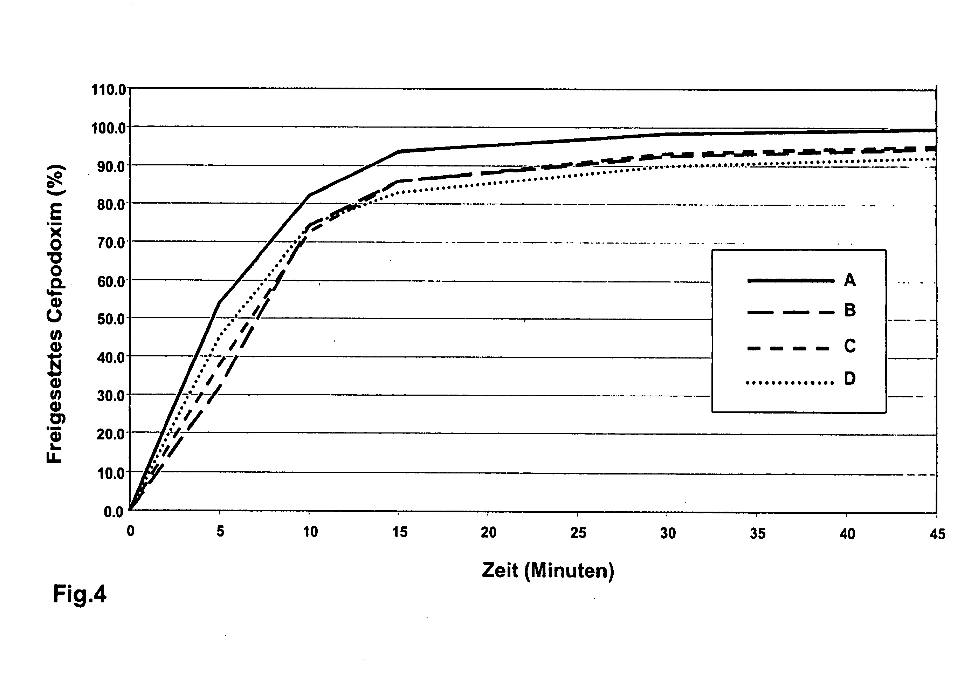

Die Freisetzung des Cefpodoxims aus den beschichteten Pellets wurde nach der in Beispiel 1 angegebenen Methode jeweils in 1000 ml Na-Phosphat-Puffer, pH 5,0 bei 75 U min⁻¹ über eine Dauer von 60 Minuten bestimmt. Das Ergebnis zur Freisetzung von Cefpodoxim aus den Pelletkernen, welche mit unterschiedlichen Mengen der Carnaubawachs/Xanthangummi-Mischung beschichtet sind, ist in Figur 4 abgebildet.

Wie aus Figur 4 ersichtlich ist, beeinflusst die aufgetragene Überzugsmenge aus Carnaubawachs und Hydrogelbildner (B) das Freisetzungsprofil nur unwesentlich. (A) 20 Gew. % Wachsauftrag, (B) 15 Gew. % Wachsauftrag, (C) 10 Gew. % Wachsauftrag, (D) 5 Gew. % Wachsauftrag.

### Beispiel 3: (Cefpodoxim Pellets beschichtet mit Carnaubawachs und verschiedenen Hydrogelbildnern (B))

Cefpodoxim 100 mg - Granulat zur Herstellung einer oralen Suspension im Trinkhalm (geschmacksmaskierte Pellets).

Die Cefpodoxim-Granulate wurden analog zum Verfahren nach Beispiel 1 hergestellt. Die Mengen der jeweiligen Komponenten aus Beispiel 1 (80 mg Cefpodoxim) wurden an die 100 mg Darreichungsformen entsprechend angepasst. Die einzelnen Zusammensetzungen der Beschichtung mit Carnaubawachs und Hydrogelbildner (B) sind in nachfolgender Tabelle zusammengefasst:

| lipophile Komponente (A) | Hydrogelbildner (B) | Auftrag Überzug |
|---|---|---|
| 75% Carnaubawachs | 15% L-HPC | 10% g/g |
| 75% Carnaubawachs | 15% Guargummi | |
| 75% Carnaubawachs | 15% Xanthangummi | |
| 75% Carnaubawachs | 15% Carbomer | |

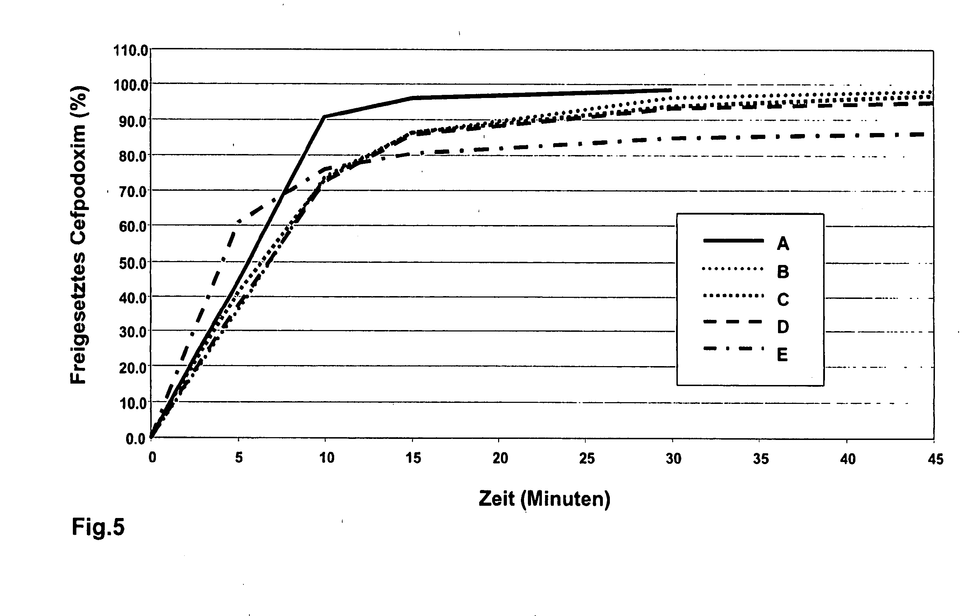

Die Freisetzung des Cefpodoxims aus den beschichteten Pellets wurde nach der in Beispiel 1 angegebenen Methode jeweils in 1000 ml Na-Phosphat-Puffer, pH 5,0 bei 75 U min⁻¹ über eine Dauer von 60 Minuten bestimmt. Das Ergebnis zur Freisetzung von Cefpodoxim aus den Pelletkernen, welche mit Carnaubawachs und verschiedenen Hydrogelbildnern (B) beschichtet sind, ist in Figur 5 abgebildet.

Wie aus Figur 5 hervorgeht, ist bei verschiedenen Hydrogelbildnern (B) die Freisetzung von Cefpodoxim nicht wesentlich verändert. (A): unbeschichtete Cefpodoxim-Pellets (Vergleichsbeispiel 5); (B): beschichtet mit 10 Gew.-% Carnaubawachs + L-HPC LH21; (C): beschichtet mit 10 Gew.-% Carnaubawachs + Meyprodor 400; (D): beschichtet mit 10 Gew.-% Carnaubawachs + Xanthangummi; (E): beschichtet mit 9 Gew.-% Carnaubawachs + Carbomer Typ 980.

### Beispiel 4: (Bioverfügbarkeit - beschichtete Cefpodoxim Pellets vs. Saft)

Es wurden 100 mg Cefpodoxim Pellets gemäß folgender Zusammensetzung hergestellt:

| | |
|---|---|
| Cefpodoxim proxetil, USP. entsprechend | 130,45 mg |
| Cefpodoxim | 100,00 mg |
| Carrageenan, NF | 52,50 mg |
| Calciumphosphat, Ph.Eur. | 26,55 mg |
| Saccharoseester, Typ S-1570, E473 | 10,50 mg |
| | |
| Carnaubawachs, Ph.Eur. | 18,70 mg |
| Xanthan, Ph.Eur | 3,30 mg |
| | 242,00 mg |

Die Herstellung der Cefpodoxim Pellets und deren Beschichtung mit Carnaubawachs erfolgte analog Beispiel 1.

Klinische Studie:
- offen, Einzeldosis, Zwei-Behandlungen, Zwei-Zeiträume, Zwei-Sequenzen, Cross-Over;
- 24 gesunde, männliche Kaukasier im Alter von 18 bis 45 Jahren; mit einem Körpergewicht entsprechend einem *Body Mass Index* von 18 bis 29 kg/m²; einem systolischen Blutdruck von 90 bis 145 mmHg und einem diastolischen Blutdruck von 65 bis 89 mmHg gemessen nach 5 min in Rückenlage; EKG ohne auffälligen Befund;
- *fed administration,* d.h. während der Einnahme eines *high fed = American Standard Breakfast.*

Es wurde die Plasmakonzentration bei Verabreichung der beschichteten Pellets und die Plasmakonzentration bei Verabreichung einer Saftformulierung gleicher Dosis (Orelox® junior, Deutschland) gemessen. Aus dem Plasmakonzentrations-Zeit-Diagramm wurden die gemittelten pharmakokinetischen Parameter Cₘₐₓ und AUC berechnet.

Der Vergleich dieser Werte für die erfindungsgemäßen Pellets mit der Saftformulierung des Standes der Technik ergab, dass die erfindungsgemäßen Pellets zu der Saftformulierung bioäquivalent sind (90% Konfidenzintervall (CI) innerhalb des Akzeptanzintervalls 80%-125%):

| | Quotient [%] (90% CI) (A/B) |
|---|---|
| Cₘₐₓ | 86 (82 ; 91) |
| AUC_{(0-∞)} | 93 (89 ; 96,7) |

A: Cefpodoximproxetil - beschichtete Pellets (100 mg Cefpodoxim)
B: Cefpodoximproxetil - Orelox® Junior-Suspension (12,5 ml enthalten 100 mg Cefpodoxim)

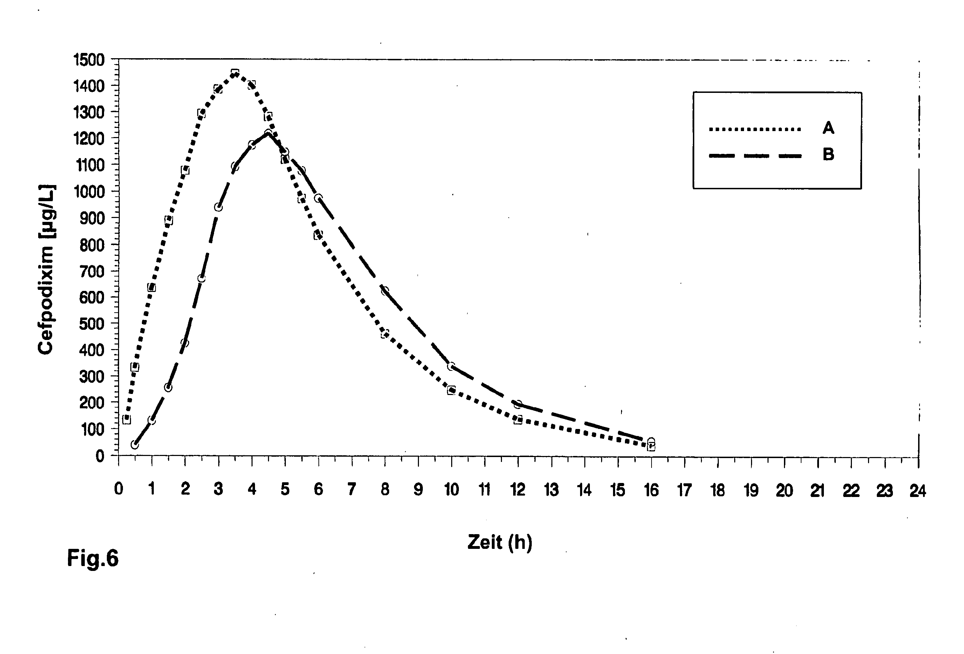

Die Plasmakonzentrations-Zeit-Diagramme (Mittelwertskurven) sind in Figur 6 abgebildet.

### Beispiel 5: Bioverfügbarkeit - unbeschichtete Cefpodoxim Pellets vs. beschichtete Cefpodoxim Pellets vs. Saft (Orelox^{®} junior 10 ml)

Es wurde analog Beispiel 4 die Plasmakonzentration bei Verabreichung unbeschichteter Cefpodoxim Pellets (vgl. Vergleichsbeispiel 5), die Plasmakonzentration bei Verabreichung der erfindungsgemäß beschichteten Cefpodoxim Pellets (vgl. Beispiel 1) und die Plasmakonzentration bei Verabreichung mit Eudragit/Zitronensäure beschichteter Cefpodoxim Pellets (vgl. Vergleichsbeispiel 6) bei jeweils gleicher Dosis gemessen (80 mg Cefpodoxim) bei Einnahme mit einem *high fed = American Standard Breakfast* gemäß Guideline.

Aus dem Plasmakonzentrations-Zeit-Diagramm wurden die gemittelten pharmakokinetischen Parameter Cₘₐₓ und AUC berechnet und jeweils durch Quotientenbildung mit der Saftformulierung (Referenz) verglichen:

| Cefpodoximproxetil | Parameter | Quotient [%], (Tₓ/Referenz) | 90% CI |
|---|---|---|---|
| T1 | Cmax | 89 | [76 ; 103] |
| | AUC_{(o-inf)} | 94 | [86 ; 103] |
| T2 | Cmax | 98 | [84 ; 114] |
| | AUC_{(o-inf)} | 99 | [91 ; 108] |
| T3 | Cmax | 47 | [40 55] |
| | AUC_{(0-inf)} | 63 | [57 ; 69] |

Wie aus den Daten ersichtlich ist, liegen sowohl die Werte der unbeschichteten Pellets (T1) als auch der erfindungsgemäßen beschichteten Pellets (T2) innerhalb des 90% Konfidenzintervalles (90% CI), d.h. sie sind bioäquivalent zur Referenz-Saftformulierung. Die mit Eudragit + Zitronensäure beschichteten Pellets (T3) sind jedoch nicht bioäquivalent zur Referenz-Saftformulierung, obwohl sie bei pH 6,4 *(fasted)* und bei pH 5,0 mit 0,1% SLS ein *in vitro* Freisetzungsverhalten zeigen, welches mit den Formulierungen T1 und T2 durchaus vergleichbar ist. So ließ sich nur aus dem in-vitro-Verhalten der Formulierung bei pH 5,0 ohne SLS dieses *in-vivo-*Verhalten vorhersagen, wobei jedoch eigentlich eine dem 0,1 % SLS Zusatz analoge Solubilisierung durch die erhöhte Gallensäurekonzentration *in vivo* nach Nahrungsaufnahme hätte erwartet werden können.

### Beispiel 6: Wirksamkeit der Geschmacksmaskierung (electronic tongue)

Durch Löslichkeitsstudien wurde die Wirksamkeit der Beschichtung der erfindungsgemäßen Pellets als Geschmacksmaskierung untersucht. Dazu wurden erfindungsgemäße Cepodoxim Proxetil Pellets mit Hilfe eines *Solid Form Dissolution Analyzer* und geeigneter Sensoren potentiometrisch, multioranoleptisch im Vergleich zu einer Placebo-Formulierung bzw. unüberzogenen Wirkstoffpellets untersucht. Der Durchfluss betrug 10 ml/min, die Testzeit 100 s, die Aquisitionszeit 550 sec., das Gefäßvolumen 160 ml.

Die Dosierung von Cefpodoxim Proxetil entsprach jeweils 100 mg:

| Beschichtung | Pelletmenge |
|---|---|
| unbeschichtet | 219 mg |
| 5% | 230 mg |
| 10% | 239 mg |
| 15% | 252 mg |
| 20% | 263 mg |

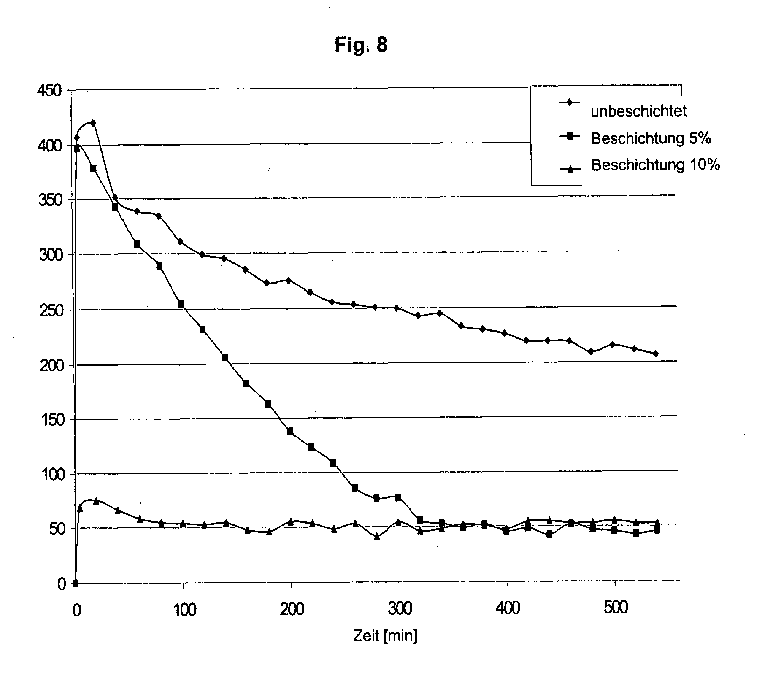

Die ausgewerteten Messkurven der potentiometrischen Untersuchungen nähern sich mit zunehmender Menge an Überzug stetig der Messkurve für die Placeboformulierung an. Die Messergebnisse sind in Figur 8 dargestellt.

Bei einer Beschichtungsmenge ab ca. 10% ist unter den gewählten Bedingungen praktisch kein Unterschied mehr zur Placebo-Formulierung zu erkennen, so dass unter diesen Bedingungen kein bitterer Geschmack mehr detektiert werden kann.

## Patentansprüche

1. Beschichtete Pellets, welche einen in Wasser schwerlöslichen pharmazeutischen Wirkstoff enthalten, unter *in vitro* Bedingungen in Phosphat-Puffer bei pH 5,0 nach 30 Minuten zumindest 80% des Wirkstoffs freisetzen und
mit einer Zusammensetzung beschichtet sind, die ein Wachs (A) und einen Hydrogelbildner (B) umfasst, wobei das Wachs (A)
(i) einen HLB-Wert ≤ 5, und/oder
(ii) einen Schmelzbereich ≥ 60°C, und/oder
(iii) einen Erstarrungsbereich Δ von weniger als 35°C, und/oder
(iv) eine Dichte ≥ 0,80 g cm⁻³
aufweist,
wobei der schwerlösliche Wirkstoff bei 23 °C in reinem Wasser eine Löslichkeit von höchstens 20 mg ml⁻¹ aufweist und ein Antibiotikum ist, welches
- gegen gram⁺ und/oder gegen gram⁻ Bakterien und/oder
- bakteriostatisch, bakterizid und/oder bakteriolytisch
wirksam ist.

2. Pellets nach Anspruch 1, **dadurch gekennzeichnet, dass** der Hydrogelbildner (B) ausgewählt ist aus der Gruppe bestehend aus Alginsäure, Alginat, amidiertem Pektin, Propylenglycolalginat, Na-Carboxymethylcellulose, Carbomer, Chitosan, Dammar-Gummi, Dextrinen, Furcellaran, Guargummi, Guarkernmehl, Gellan, Ghatti-Gummi, Gummi arabicum, Gummi aus Fichtensaft, Johannisbrotkernmehl, Karayagummi, Keratin, Konjakmehl, L-HPC, Locust Bean Gum, Mastix, Pektin, Tarakemmehl, Traganth und Xanthangummi.

3. Pellets nach einem der vorstehenden Ansprüche, **dadurch gekennzeichnet, dass** es sich um Extrusionspellets handelt.

4. Pellets nach einem der vorstehenden Ansprüche, **dadurch gekennzeichnet, dass** sie im Kern einen Zuckerester und/oder ein sulfatisiertes Polysaccharid und ggf. Ca₃(PO₄)₂ enthalten.

5. Pellets nach Anspruch 4, **dadurch gekennzeichnet, dass** das sulfatisierte Polysaccharid Carrageenan ist.

6. Pellets nach einem der vorstehenden Ansprüche, **dadurch gekennzeichnet, dass** der Gewichtsanteil des Hydrogelbildners (B) am Gesamtgewicht des Filmüberzugs im Bereich von 2,5 bis 50 Gew.-% liegt.

7. Pellets nach einem der vorstehenden Ansprüche, **dadurch gekennzeichnet, dass** sie unter *in vitro* Bedingungen in Phosphatpuffer bei pH 5,0 nach 30 Minuten zumindest 90% des Wirkstoffs freisetzen.

8. Pellets nach einem der vorstehenden Ansprüche, **dadurch gekennzeichnet, dass** das Antibiotikum ein Zellwandsynthesehemmer und/oder ein Hemmer der Proteinbiosynthese am Ribosom und/oder ein Gyrase-Hemmer und/oder ein Folsäureantagonist und/oder ein Hemmer der bakteriellen RNA-Polymerase ist.

9. Pellets nach Anspruch 8, **dadurch gekennzeichnet, dass** das Antibiotikum ausgewählt ist aus der Gruppe bestehend aus Tetracyclinen [ATCJ01A], Amphenicolen [ATCJ01 B], ß-Lactam-Antibiotika, Penicillinen [ATCJ01 C], anderen ß-Lactam-Antibiotika [ATCJ01 D], Sulfonamiden und Trimethoprim [ATCJ01 E], Macroliden, Lincosamiden und Streptograminen [ATCJ01 F], Aminoglycosid-Antibiotika [ATCJ01 G], Chinolonen [ATCJ01M] und anderen Antibiotika [ATCJ01X].

10. Pellets nach Anspruch 9, **dadurch gekennzeichnet, dass** das Antibiotikum ausgewählt ist aus der Gruppe bestehend aus Cephalosporinen der 2. Generation [ATCJ01 DC], Cephalosporinen der 3. Generation [ATCJ01 DD] und Makroliden [ATCJ01 FA].

11. Pellets nach Anspruch 9 oder 10, **dadurch gekennzeichnet, dass** das Antibiotikum ausgewählt ist aus der Gruppe bestehend aus Azithromycin, Cefpodoxim, Cefpodoximproxetil, Cefuroxim, Cefuroximaxetil, Cefixim, Cefdinir und Clarithromycin.

12. Orale Darreichungsform umfassend Pellets nach einem der vorstehenden Ansprüche.

13. Pellets nach einem der Ansprüche 1 bis 11 oder Darreichungsform nach Anspruch 12, **dadurch gekennzeichnet, dass** nach oraler Verabreichung die maximale Plasmakonzentration Cₘₐₓ des Wirkstoffs nach tₘₐₓ im Bereich von 1 h bis 8 h erreicht wird.

14. Verwendung eines Antibiotikums definiert wie in einem der Ansprüche 1 oder 8 bis 10 zur Herstellung von Pellets bzw. einer Darreichungsform nach einem der Ansprüche 1 bis 13 zur Vorbeugung und/oder Behandlung von bakteriellen Erkrankungen.

15. Trinkhalm enthaltend Pellets bzw. eine Darreichungsform nach einem der Ansprüche 1 bis 13.

## Claims

1. Coated pellets comprising a pharmaceutical active agent that is hardly soluble in water, releasing at least 80% of the active agent under *in vitro* conditions in a phosphate buffer at a pH value of 5.0 after 30 minutes, and which
are coated with a composition comprising a wax (A) and a hydrogel former (B), wherein the wax (A) has
(i) an HLB value of ≤ 5, and/or
(ii) a melting range of ≥ 60°C, and/or
(iii) a solidification range Δ of less than 35°C, and/or
(iv) a density of ≥ 0.80 g cm⁻³,
wherein the hardly soluble active agent has a solubility in pure water at 23°C of not more than 20 mg m⁻¹ and is an antibiotic, which is effective
- against gram⁺ and/or against gram⁻ bacteria, and/or
- bacteriostatic, bactericide, and/or bacteriolytic.

2. The pellets according to claim 1, **characterized in that** the hydrogel former (B) is selected from the group consisting of alginic acid, alginate, amidated pectin, propylene glycol alginate, Na-carboxy methyl cellulose, carbomer, chitosan, dammar gum, dextrins, furcellaran, guar gum, guar flour, gellane, ghatti gum, gum arabic, gum from spruce juice, St John's bread core meal, karaya gum, keratin, conjac meal, L-HPC, locust bean gum, mastic, pectin, tara core meal, tragacanth, and xanthan gum.

3. The pellets according to one of the previous claims, **characterized in that** the same are extrusion pellets.

4. The pellets according to one of the previous claims, **characterized in that** the same contain sugar ester and/or a sulphated polysaccharide, and possibly Ca₃(PO₄)₂ within the core.

5. The pellets according to claim 4, **characterized in that** the sulphated polysaccharide is carrageenan.

6. The pellets according to one of the previous claims, **characterized in that** the proportion of weight of the hydrogel former (B) of the total weight of the film coating is within the range of 2.5 to 50 wt.-%.

7. The pellets according to one of the previous claims, **characterized in that** the same releases at least 90% of the active agent under *in vitro* conditions in a phosphate buffer at a pH value of 5.0 after 30 minutes.

8. The pellets according to one of the previous claims, **characterized in that** the antibiotic is a cell wall synthesis inhibitor and/or an inhibitor of the protein biosynthesis at the ribosome, and/or a gyrase inhibitor, and/or a folic acid antagonist, and/or an inhibitor of the bacterial RNA polymerase.

9. The pellets according to claim 8, **characterized in that** the antibiotic is selected from the group consisting of teracyclins [ATCJ01A], amphenicoles [ATCJ01B], β-lactam antibiotics, penicillins [ATCJ01C], other β-lactam antibiotics [ATCJ01D], sulfonamides, and trimethoprim [ATCJ01E], macrolides, lincosamides, and streptogramines [ATCJ01F], aminoglycoside antibiotics [ATCJ01G], chinolons [ATCJ01M] as well as other antibiotics [ATCJ01X].

10. The pellets according to claim 9, **characterized in that** the antibiotic is selected from the group consisting of cephalosporins of the 2^{nd} generation [ATCJ01 DC], cephalosporins of the 3^{rd} generation [ATCJ01 DD], and macrolides [ATCJ01 FA].

11. The pellets according to claims 9 or 10, **characterized in that** the antibiotic is selected from the group consisting of azithromycin, cefpodoxim, cefpodoximproxetil, cefuroxim, cefuroximaxetil, cefixim, cefdinir, and clarithromycin.

12. An oral pharmaceutical form comprising pellets according to one of the previous claims.

13. The pellets according to one of the claims 1 to 11, or the pharmaceutical form according to claim 12, **characterized in that** after oral administration the maximum plasma concentration Cₘₐₓ of the active agent after tₘₐₓ is achieved within a range of 1 hr to 8 hrs.

14. Use of an antibiotic as defined in one of the claims 1 or 8 to 10 for producing pellets, or a pharmaceutical form according to one of the claims 1 to 13 for preventing and/or treating bacterial diseases.

15. A drinking straw comprising pellets, or a pharmaceutical form, respectively, according to one of the claims 1 to 13.

## Revendications

1. Pellets enrobés qui contiennent un principe actif pharmaceutique difficilement soluble dans l'eau, dégageant au bout de 30 minutes, sous des conditions *in-vitro,* au moins 80% du principe actif dans un tampon de phosphate à pH 5,0, et
qui sont revêtus d'une composition qui comprend une cire (A) et un générateur d'hydrogel (B), sachant que la cire (A) présente
(i) une valeur HLB ≤ 5, et/ou
(ii) une plage de fusion ≥ 60 °C, et/ou
(iii) une plage de solidification Δ de moins de 35 °C, et/ou
(iv) une densité ≥ 0,80 g/cm3,
le principe actif difficilement soluble présentant à 23 °C dans l'eau pure une solubilité de tout au plus 20 mg/ml⁻¹ et étant un antibiotique, qui
- est efficace contre des bactéries gram⁺ et/ou gram⁻ et/ou
- est bactériostatique, bactéricide et/ou bactériolytique.

2. Pellets selon la revendication 1, **caractérisés en ce que** le générateur d'hydrogel (B) est choisi parmi le groupe composé de l'acide alginique, de l'alginate, de la pectine amidée, de l'alginate de propylène glycol, de la Na-carboxyméthylcellulose, de l'acide polyacrylique, du chitosane, de la gomme de dammar, des dextrines, du furcellarane, de la gomme de guar, de la farine de guar, du gellan, de la gomme ghatti, de la gomme arabique, de la gomme de sève d'épicéa, de la farine de caroube, de la gomme de karaya, de la kératine, de la farine de konjak, du L-HPC, de la Locust Bean Gum, du Mastix, de la Pectine, de la farine de tara, du traganth et de la gomme de xanthane.

3. Pellets selon l'une quelconque des revendications précédentes, **caractérisés en ce qu'**il s'agit de pellets extrudés.

4. Pellets selon l'une quelconque des revendications précédentes, **caractérisés en ce que** qu'ils contiennent dans le coeur un ester de sucre et/ou un polysaccharide sulfaté et le cas échéant du Ca₃(PO₄)₂.

5. Pellets selon la revendication 4, **caractérisés en ce que** le polysaccharide sulfaté est de la carraghénane.

6. Pellets selon l'une quelconque des revendications précédentes, **caractérisés en ce que** la part pondérale du générateur d'hydrogels (B) au poids total du film de revêtement est comprise dans l'intervalle de 2,5 à 50 % en poids.

7. Pellets selon l'une quelconque des revendications précédentes, **caractérisés en ce qu'**au bout de 30 minutes et sous des conditions *in-vitro,* ils dégagent au moins 90% de principe actif dans les tampons de phosphate à pH 5,0.

8. Pellets selon l'une quelconque des revendications précédentes, **caractérisés en ce que** l'antibiotique est un inhibiteur de synthèse de membrane cellulaire et/ou un inhibiteur de la biosynthèse des protéines sur le ribosome et/ou un inhibiteur de gyrase et/ou un antagoniste de l'acide folique et/ou inhibiteur de l'ARN polymérase bactérienne.

9. Pellets selon la revendication 8, **caractérisés en ce que** l'antibiotique est choisi parmi le groupe composé des tétracyclines [ATCJ01A], des amphénicols [ATCJ01B], des antibiotiques à β-lactame, des pénicillines [ATCJ01C], d'autres antibiotiques à β-lactame [ATCJ01D], des sulfonamides et du triméthoprim [ATCJ01E], des macrolides, des lincosamides et des streptogramines [ATCJ01F], des antibiotiques à aminoglycosides [ATCJ01G], des quinolones [ATCJ01M] et d'autres antibiotiques [ATCJ01X].

10. Pellets selon la revendication 9, **caractérisés en ce que** l'antibiotique est choisi parmi le groupe composé des céphalosporines de la 2e génération [ATCJ01DC], des céphalosporines de la 3e génération [ATCJ01DD] et des macrolides [ATCJ01FA].

11. Pellets selon la revendication 9 ou 10, **caractérisés en ce que** l'antibiotique est choisi parmi le groupe composé de l'azithromycine, de la cefpodoxime, de la cefpodoxime proxétil, de la céfuroxime, du céfuroxime axétil, de la céfixime, du cefdinir et de la clarithromycine.

12. Forme d'administration orale comprenant des pellets selon l'une quelconque des revendications précédentes.

13. Pellets selon l'une quelconque des revendications 1 à 11 ou forme d'administration selon la revendication 12, **caractérisés en ce qu'**après l'administration orale, la concentration maximale cₘₐₓ du plasma en principe actif est atteinte au bout de tₘₐₓ dans l'intervalle de 1 h à 8 h.

14. Utilisation d'un antibiotique défini comme dans l'une quelconque des revendications 1 ou 8 à 10 à la fabrication de pellets, respectivement d'une forme d'administration selon l'une quelconque des revendications 1 à 13 à la prévention et/ou au traitement de maladies bactériennes.

15. Paille à boire contenant des pellets, respectivement une forme d'administration selon l'une quelconque des revendications 1 à 13.
